# EUROPEAN PATENT SPECIFICATION

(11) **EP 2 580 595 B1**
(45) Date of publication and mention of the grant of the patent: **21.10.2015**
(21) Application number: 11722838.7
(22) Date of filing: 03.06.2011
(51) Int. Cl.: G01N 33/68

(54) **DIAGNOSTIC AND PROGNOSTIC METHOD FOR HUMAN TAUOPATHIES**
VERFAHREN ZUR PROGNOSE ODER DIAGNOSE VON TAUOPATHIEN
PROCEDE DE DIAGNOSTIC ET DE PRONOSTIC DE PATHOLOGIES TAU

(30) Priority: 11.06.2010 IT RM20100320
(43) Date of publication of application: 17.04.2013
(73) Proprietor: Consiglio Nazionale Delle Ricerche, 00185 Roma (IT); Fondazione European Brain Research Institute (EBRI), 00143 Roma (IT)
(72) Inventor: AMADORO, Giuseppina, I-00144 Roma (RM) (IT); CALISSANO, Pietro, I-00199 Roma (RM) (IT); CORSETTI, Veronica, I-00040 Pomezia (RM) (IT)
(74) Representative: Capasso, Olga
(86) International application number: PCT/EP2011/059206
(87) International publication number: WO 2011/154321

(56) References cited:
- WO-A1-02/062851
- AMADORO GIUSEPPINA ET AL: "A NH2 tau fragment targets neuronal mitochondria at AD synapses: possible implications for neurodegeneration", JOURNAL OF ALZHEIMER'S DISEASE, IOS PRESS, AMSTERDAM, NL, vol. 21, no. 2, 21 June 2010 (2010-06-21), pages 445-470, XP008132930, ISSN: 1387-2877
- CORSETTI V ET AL: "Identification of a caspase-derived N-terminal tau fragment in cellular and animal Alzheimer's disease models", MOLECULAR AND CELLULAR NEUROSCIENCES, SAN DIEGO, US, vol. 38, no. 3, 1 July 2008 (2008-07-01), pages 381-392, XP022777376, ISSN: 1044-7431, DOI: DOI:10.1016/J.MCN.2008.03.011 [retrieved on 2008-04-10]
- JOHNSON G V ET AL: "The tau protein in human cerebrospinal fluid in Alzheimer's disease consists of proteolytically derived fragments.", JOURNAL OF NEUROCHEMISTRY JAN 1997 LNKD- PUBMED:8978756, vol. 68, no. 1, January 1997 (1997-01), pages 430-433, XP008132995, ISSN: 0022-3042
- KIM WONHEE ET AL: "Secretion of human tau fragments resembling CSF-tau in Alzheimer's disease is modulated by the presence of the exon 2 insert.", FEBS LETTERS 16 JUL 2010 LNKD- PUBMED:20553717, vol. 584, no. 14, 27 May 2010 (2010-05-27) , pages 3085-3088, XP002623017, ISSN: 1873-3468
- HANES JOZEF ET AL: "Rat tau proteome consists of six tau isoforms: implication for animal models of human tauopathies.", JOURNAL OF NEUROCHEMISTRY MAR 2009, vol. 108, no. 5, March 2009 (2009-03), pages 1167-1176, ISSN: 1471-4159
- JANKE C ET AL: "Phylogenetic diversity of the expression of the microtubule-associated protein tau: implications for neurodegenerative disorders.", BRAIN RESEARCH. MOLECULAR BRAIN RESEARCH 7 MAY 1999, vol. 68, no. 1-2, 7 May 1999 (1999-05-07), pages 119-128, ISSN: 0169-328X
- TAKUMA HIROSHI ET AL: "Isoforms changes of tau protein during development in various species.", BRAIN RESEARCH. DEVELOPMENTAL BRAIN RESEARCH 14 MAY 2003, vol. 142, no. 2, 14 May 2003 (2003-05-14), pages 121-127, ISSN: 0165-3806
- MCMILLAN PAMELA ET AL: "Tau isoform regulation is region- and cell-specific in mouse brain.", THE JOURNAL OF COMPARATIVE NEUROLOGY 20 DEC 2008, vol. 511, no. 6, 20 December 2008 (2008-12-20), pages 788-803, ISSN: 1096-9861

### SEQUENCE LISTING

<110> Consiglio Nazionale Delle Ricerche et al.
<120> DIAGNOSTIC AND PROGNOSTIC METHOD FOR HUMAN TAUOPATHIES
<130> PCT 112327
<160> 3
<170> PatentIn version 3.5
<210> 1
   <211> 205
   <212> PRT
   <213> Homo sapiens
<400> 1
   Gln Gly Gly Tyr Thr Met His Gln Asp Gln Glu Gly Asp Thr Asp Ala
   1               5                   10                  15
   Gly Leu Lys Glu Ser Pro Leu Gln Thr Pro Thr Glu Asp Gly Ser Glu
               20                  25                  30
   Glu Pro Gly Ser Glu Thr Ser Asp Ala Lys Ser Thr Pro Thr Ala Glu
           35                  40                  45
   Asp Val Thr Ala Pro Leu Val Asp Glu Gly Ala Pro Gly Lys Gln Ala
       50                  55                  60
   Ala Ala Gln Pro His Thr Glu Ile Pro Glu Gly Thr Thr Ala Glu Glu
   65                  70                  75                  80
   Ala Gly Ile Gly Asp Thr Pro Ser Leu Glu Asp Glu Ala Ala Gly His
                   85                  90                  95
   Val Thr Gln Ala Arg Met Val Ser Lys Ser Lys Asp Gly Thr Gly Ser
               100                 105                 110
   Asp Asp Lys Lys Ala Lys Gly Ala Asp Gly Lys Thr Lys Ile Ala Thr
           115                 120                 125
   Pro Arg Gly Ala Ala Pro Pro Gly Gln Lys Gly Gln Ala Asn Ala Thr
       130                 135                 140
   Arg Ile Pro Ala Lys Thr Pro Pro Ala Pro Lys Thr Pro Pro Ser Ser
   145                 150                 155                 160
   Gly Glu Pro Pro Lys Ser Gly Asp Arg Ser Gly Tyr Ser Ser Pro Gly
                   165                 170                 175
   Ser Pro Gly Thr Pro Gly Ser Arg Ser Arg Thr Pro Ser Leu Pro Thr
               180                 185                 190
   Pro Pro Thr Arg Glu Pro Lys Lys Val Ala Val Val Arg
           195                 200                 205
<210> 2
   <211> 14
   <212> PRT
   <213> Homo sapiens
<400> 2
   Asp Arg Lys Asp Gln Gly Gly Tyr Thr Met His Gln Asp Gln
   1               5                   10
<210> 3
   <211> 298
   <212> PRT
   <213> Homo sapiens
<400> 3

## Description

### FIELD OF THE INVENTION

The present invention relates to a method for the diagnosis and/or prognosis of human tauopathies, in particular Alzheimer's disease (AD). The method is based on the detection and quantification of a 20-22 kDa NH₂-tau fragment in CSF (CerebroSpinal Fluid).

### BACKGROUND ART

Synapses are ultrastructural sites for memory storage in brain and synaptic damage is the best pathologic correlate of cognitive decline in Alzheimer's disease (AD). Post-translational hyperphosphorylation, enzyme-mediated truncation, conformational modifications and aggregation of tau protein into neurofibrillary tangles (NFTs) are hallmarks for an heterogenous group of neurodegenerative disorders, so-called tauopathies. AD is a secondary tauopathy since it is pathologically distinguished by the presence of β-amyloid-containing senile plaques and the presence of tau-positive NFTs in the neocortex and hippocampus.

Alzheimer's disease is a progressive neurodegenerative dementia characterized by memory deficits and extensive neuronal loss. Although the presence of extracellular β-amyloid plaques and intracellular tau neurofibrllary tangles are typically considered classical neuropathological hallmarks of such disorder, loss of synapses in selected brain areas devoted to memory and learning processes better correlates to cognitive decline than plaques and tangles [1-6]. Indeed, synaptic decay is the earliest sign of cognitive impairment occurring before or even in the absence of neuronal loss both in patients and in transgenic mouse models of AD [5-14]. Postmortem studies using quantitative electron microscopy (EM) and biochemical analyses in early-to-mid AD patients have reported loss of axon terminals [15,16], altered expression of pre- and post-synaptic proteins [17-22] and disappearance of dendritic spines, which are known to be the major postsynaptic site for excitatory synaptic transmission [23-26].

Several studies from cellular and animal models suggest that tau protein is a necessary mediator of Aβ-induced neurotoxicity [27-29]. Aβ intraneuronal accumulation precedes and promotes neurofibrillary tau pathology in transgenic mice and in AD brains [30-34], probably also by the generation of truncated tau fragments [35-38] that may acquire toxicity properties, regardless of their aggregation state [39]. Increased level of NH₂-truncated tau forms are early detected in cerebrospinal fluid of AD patients [40,41] and tau proteolytic cleavage generates amyloidogenic fragments, which are more prone to aggregation [36,37,42]. On the other hand, Aβ deposition and tau hyperphosphorylation both develop near to each other in synaptic terminals of hippocampus and enthorinal cortex from AD subjects and disease-linked transgenic mice, suggesting that amyloid and tau pathologies are *in vivo* spatially associated in-neuronal synapses [43,44].

An immunohistochemical analysis performed with a Caspase-Cleaved Protein-NH₂ tau antibody (termed CCP-NH₂ tau antibody) [35]-recognizing the sequence of human tau protein located at the amino-terminal end DRKD₂₅-QGGYTMHQDQ known to be one of the consensus sites for cleavage by caspase(s) [45]- shows a selective and widespread labeling of NFTs, neuropil threads and dystrophic neurites in AD brain sections, but not in age-matched controls. By Western blotting and imunohistochemistry studies, the authors have also demonstrated that a 20-22 kDa NH₂- tau fragment- which is likely found between 26 and 230 aminoacids of the longest full length tau isoform (NCBI *protein* ID AAC04279.1), thus having the following sequence: QGGYT MHQDQEGDTD AGLKESPLQT PTEDGSEEPGSETSDAKSTP TAEDVTAPLV DEGAPGKQAA AQPHTEIPEG TTAEEAGIGD TPSLEDEAAG HVTQARMVSK SKDGTGSDDK KAKGADGKTK IATPRGAAPP GQKGQANATR IPAKTPPAPK TPPSSGEPPK SGDRSGYSSP GSPGTPGSRS RTPSLPTPPT REPKKVAVVR (SEQ ID No. 1) is detected in cellular and animal AD models [46]. Moreover a synthesized NH₂-26-44 peptide, which is the minimal active moiety retaining the *in vitro* deleterious effect of longer overexpressed NH₂-26-230 human tau fragment [47,48], impairs the mitochondrial oxidative phosphorylation and reduces the intracellular ATP bioavailability, probably acting on adenine nucleotide translocator (ANT)- mediated ADP/ATP exchange [49]. As also reported (Amadoro et al., 2010), the authors previously found (i) that the level of the such NH₂-tau fragment was enriched in synaptosomes and in synaptic mitochondria of human familial AD patients, compared to age- matched non-demented control samples and (ii) that it was correlated to the synaptic changes, in relation to the extent of neurofibrillary degeneration and amyloid neuropathology (iii) that it actually bound the mitochondrial ANT-1 *in vivo.*

WO02/062851 discloses a method for diagnosis/prognosis of AD and related tauopathies comprising measurements of abnormally NH₂-truncated tau using antibodies thereto. However the document does not disclose the specific NH₂-dervied tau fragment of the instant invention, nor that such specific fragment could be used for diagnosis/prognosis by its quantification in CSF.

Corsetti et al., Molec. and Cellular Neurosci. 38(3):381-392, 2008 is a study to examine in further details the role of N-terminally truncated tau in in vitro and in vivo neurodegeneration models. The document suggests the development of pharmacological treatments with selective tau caspase(s)-cleavage inhibitors to slow Alzheimer's disease (AD) progression but is silent concerning the diagnostic value of any tau fragment.

### SUMMARY OF THE INVENTION

In view of the fact that there's a direct correlation between the temporal pattering of specific CSF biomarkers and progressive neuronal atrophy in affected human CNS areas, the authors show in the present invention that the 20-22 kDa NH2-truncated tau fragment is also present in the CSF of patients carrying tauopathies, including AD, in relation to the classical neurophathological hallmarks.

Authors provide a novel, valuable, diagnostic/prognostic tool that should improve the precision level of current biological tests on CSF from patients affected by tauopathies, in particular AD.

The authors previously reported that a 20-22 kDa NH₂-truncated tau fragment was largely enriched in human mitochondria from cryopreserved synaptosomes of AD brains and that its amount in terminal fields correlated with the pathological synaptic changes and with the organelle functional impairment. This NH₂-truncated tau form was also found in other human, not AD-tauopathies, while its presence in AD patients was linked to Aβ multimeric species and to pathology severity. Native, patient-derived, Aβ (Δβ) oligomers-enriched extracts likely impaired the mitochondrial function by the *in vitro* production of 20-22 kDa NH₂- tau fragment in mature human SY5Y and in rat hippocampal neurons. This synaptic NH₂-truncated tau - whose minimal active region (i.e.NH₂26-44) *in vitro* specifically inhibited the ANT-1-mediated ADP/ATP exchange in a non-competitive manner [49] - colocalized *in vivo* with it in human AD mitochondria (Amadoro et al., 2010). Authors propose that the detection and quantification of this NH₂ -truncated tau peptide in CSF from AD patients - as well as in CSF from patients affected by other taupathies - can be used as valuable, specific pathological biomarker in clinical practice.

It is therefore an object of the present invention a method for the diagnosis and/or prognosis of a tauopathy in a subject comprising:
- quantifying the amount of a 20-22 kDa NH₂-derived tau fragment comprising the sequence QGGYT MHQDQEGDTD AGLKESPLQT PTEDGSEEPGSETSDAKSTP TAEDVTAPLV DEGAPGKQAA AQPHTEIPEG TTAEEAGIGD TPSLEDEAAG HVTQARMVSK SKDGTGSDDK KAKGADGKTK IATPRGAAPP GQKGQANATR IPAKTPPAPK TPPSSGEPPK SGDRSGYSSP GSPGTPGSRS RTPSLPTPPT REPKKVAVVR (SEQ ID No. 1) or or of a fragment of SEQ ID 1, said fragment missing 1 to 10 amino acids, in a sample of CSF obtained from the subject;
- comparing the quantified amount of the 20-22 kDa NH₂- derived tau fragment comprising SEQ ID No. 1 or of said fragment thereof in the sample to appropriate control amount.

In particular, the appropriate control amount may be the amount quantified in a normal healthy subject or in a subject non affected by a tauopathy. The method may also be used to monitor the progress of the pathology in the same subject. A tauopathy is present if in the test subject there is an increased amount of the 20-22 kDa NH2 tau fragment comprising SEQ ID No. 1 or a 20-22 kDa fragment thereof.

Preferably, the tauopathy is selected from the group of: Alzheimer's Disease (AD), Dementia with Lewy Bodies (DLB), Pick's disease (PiD), cortico basal degeneration (CBD), or progressive supranuclear palsy (PSP).

In the present invention the 20-22 kDa fragment of SEQ ID No. 1 is a smaller fragment missing approximately from 1 to 10 amino acids. The fragment may be a functional fragment of SEQ ID No. 1, i.e a fragment retaing the toxicity feature of SEQ ID No. 1.

The methods of the invention can also be used to follow the course of a tauopathy or to follow the effect of a therapeutic treatment on the tauopathy. In such case, the appropriate control amount is quantified from a subject before pharmacological treatment or at different time points.

The present invention will be now illustrated by means of the following non limiting figures.

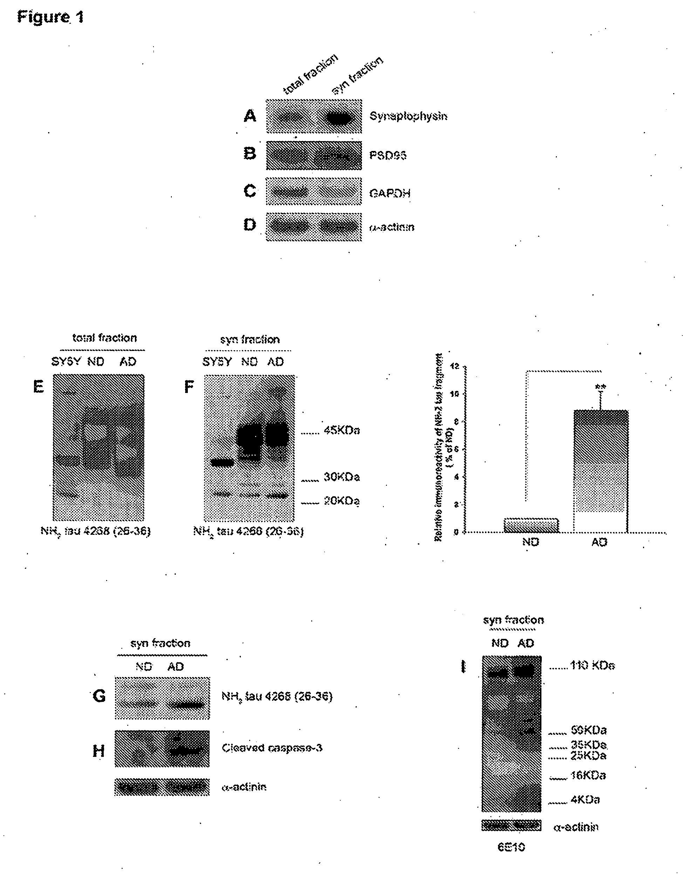

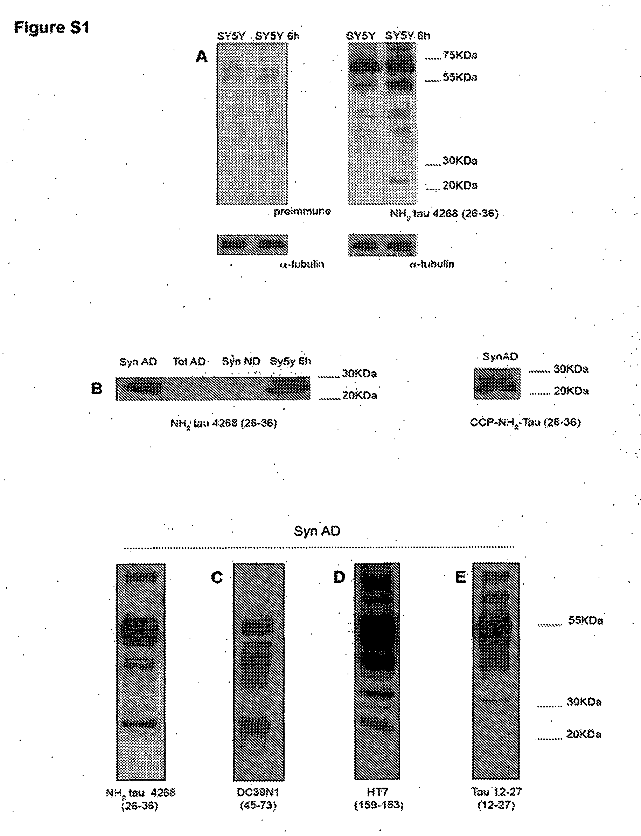

**Figure 1****.The NH₂-derived 20-22 kDa tau fragment is localized at higher level in AD synapses than in non-demented control, in correlation with the active form of caspase-3.** A-B-C-D): Quality assessment of human synaptosomes preparation. Western blotting analysis of equal proteins amounts (20 µg) prepared from total and synaptosomes (syn) fractions with antibodies specific for pre-synaptic (A) and post-synaptic proteins (B) and for the not-synaptic GAPDH protein (C) demonstrated the synaptic enrichment efficiency. α-actinin-2 was used as a loading control (D). E-F-G) Representative Western blotting (n=3) comparing equal amount of total (E) and synaptosomal (F) proteins lysates (50 µg) from non-demented control (ND) and AD subiects probed with NH₂ 4268 tau (aa26-36) antibody. In line with the authors' previous paper [46], equal amounts of total proteins extracts (50 µg) from 6h STS-treated neuronal SY5Y, which expressed highly the shortest tau isoforms [172], were also loaded for lane and included as internal control of electrophoretic mobility and of protein loading. It is noteworthy that the signal intensity of tau immunoreactivity from SY5Y total extracts is comparable in two filters, to allow direct comparison between the total protein mass applied to different lanes. In addition, since identical conditions and exposure times for image acquisition were employed for different experimental samples, the tau signal from total brain extracts became saturated in comparison with that from synaptic protein fraction. Notice also the presence of high molecular weight tau aggretates, which migrated slowly and were detectable only in pathological conditions. Densitometric analysis of 20-22 kDa NH₂ tau fragment immunoreactivity band in all diseased cases (n=15) was expressed as percentage of control sample (non-demented case) (n=10), where control has a value of 1(G). Each data point is the mean ± SE (bars) of independent experiments (n=19) and statistically significant differences were calculated by paired t-Student's test (**p<0.01). H): The synaptic distribution of cleaved caspase-3 was also checked. Representative Western blotting (n=2) comparing equal amount of synaptosomal proteins lysates (25-30 µg) from non-demented control (ND) and AD subiects probed with NH₂ 4268 tau (aa26-36) antibody and with antibody against the caspase-3 enzyme cleaved form. The protein levels were normalized to α-actinin-2 as a loading control. I): Lysates of synaptosomal (20 µg) preparations from non-demented control (ND) and AD cases, were analyzed (n=3) for 6E10 Aβ (aa4-9). α-actinin-2 was used as a loading control.

**Figure S1.Characterization of affinity and specificity of the polyclonal NH₂ 4268 tau antibody.** A-B-C-D-E): NH₂ 4268 tau antiserum (SG-4268) was generated in Sigma-Genosys (UK) laboratories against an HPLC-purified synthetic peptide, which corresponds to residues 26-36 of the longest isoform of human tau protein (project 18966-1). KLH-conjugated synthetic NH₂ 26-36 tau peptide was used for immunization of New Zealand white rabbit and test bleeds were further assessed for epitope mapping by enzyme linked immunosorbant assay (ELISA). Affinity-purified NH₂ 4268 tau antibody (0.5mg/ml) reacted with a synthetic peptide corresponding to residues 26-36 in human tau, but not with a panel of other peptides corresponding within the amino-terminal half of the protein, including peptide 1-25. The specificity of purified polyclonal antibody was next analyzed by comparing to its relative pre-immune serum, by SDS-PAGE of human protein extratcs (A). A NH₂ tau fragment of 20-22kDa molecular weight was detected only by immune serum (A left) in total protein extracts from differentiated SH-SY5Y induced to apoptosis upon 6h STS treatment, in agreement with the authors' previous report [46]. Finally, Western blotting of AD synaptosomes compared the relative immunoreactivity of NH2 4268 tau and CCP- NH2 tau antibodies. Proteins samples from AD synaptosomes were applied on the gel in two different lanes and after transfer the membrane was cut in two equal parts and analyzed in parallel with NH₂ 4268 tau and CCP- NH₂ tau antibodies (B). Notice also that the NH₂ tau fragment of 20-22 kDa molecular weight was only detected in AD synaptosomes, while it is indetectable in equivalent protein amounts from AD total extract or ND synaptosomes. β-actin was used as control loading.

The identity of the NH₂ 20-22 kDa tau truncated fragment probed with NH₂ 4268 tau was finally confirmed by Western blotting analysis of equal amounts of human synaptosomes fractions with several commercial and non-commercial phosphorylation-independent tau antibodies-i.e. Tau12-27 (aa NH₂ 12-27), DC39N1 (aa NH₂ 45-73), HT7 (aa NH₂159-163), which are directed against the proximal/middle NH₂ end of full length human tau protein, respectively. Although with different intensity, immunological affinity epitopes mapping identified the NH₂- 20-22 kDa tau fragment as positive for DC39N1 (C) and HT7 (D), while only the upper band of the doublet was positive for Tau 12-27(E), likely because of a partial destruction of epitope that could remove crucial interactions between antibody and antigen and reduce the antibody affinity. Furthermore, in agreement with the authors' previous data in cellular and animal AD models [46], Tau13 (aa NH₂ 1-13)- an antibody reacting with the extreme NH₂ end of full length human tau protein-did not visualize the NH₂ 20-22 kDa tau truncated fragment, which were instead detectable at long exposition with Tau21 (aa NH₂ 21-36) (not shown), suggesting that a removal of near part not involved in antibody binding could disrupt the conformation of adiacent molecule region necessary for the *in vivo* epitope presentation.

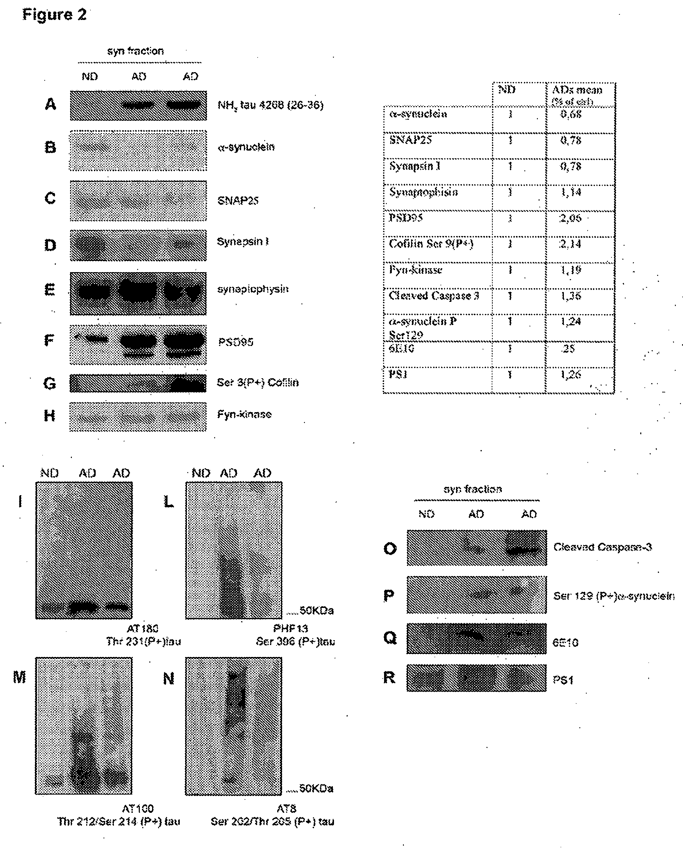

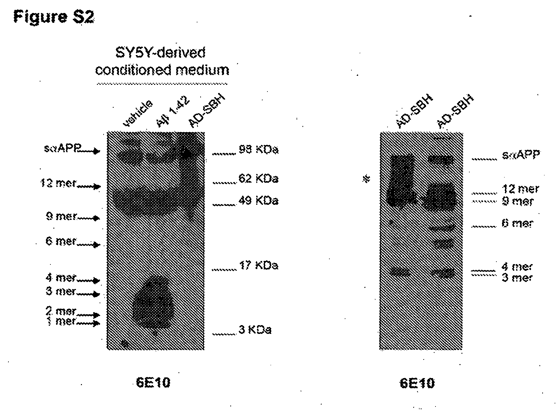

**Figure 2****. The accumulation of the NH₂-derived 20-22 kDa tau fragment is associated with synaptic alterations in AD subjects**. A-T): Immunoblotting analysis of equal proteins amounts (20 µg) of synaptosomal preparations from non-demented control (ND) and two representative AD cases (AD) for NH₂ 4268 tau (aa26-36) (A), α-synuclein (B), SNAP-25 (C), Synapsin I (D), Synaptophisin (E), PSD95 (F), Ser9 (P+) Cofilin (G), Fyn kinase (H), AT180-Thr231 (P+)tau (I), PHF13-Ser396(P+) tau(L),AT100-Thr212/Ser214(P+)tau (M), AT8-Ser202/Thr205 (P+)tau(N), cleaved caspase-3 (O), Ser129(P+)α-synuclein (P), 6E10 (Q), N-terminal PS1(R). Neither α-actinin nor β-actin (not shown)-which were used as a loading control- underwent similar changes. Densitometric analysis of bands immunoreactivity (%/β-action) detected by different antibodies was expressed as ratio of control sample (non-demented case), where control has a value of 1.

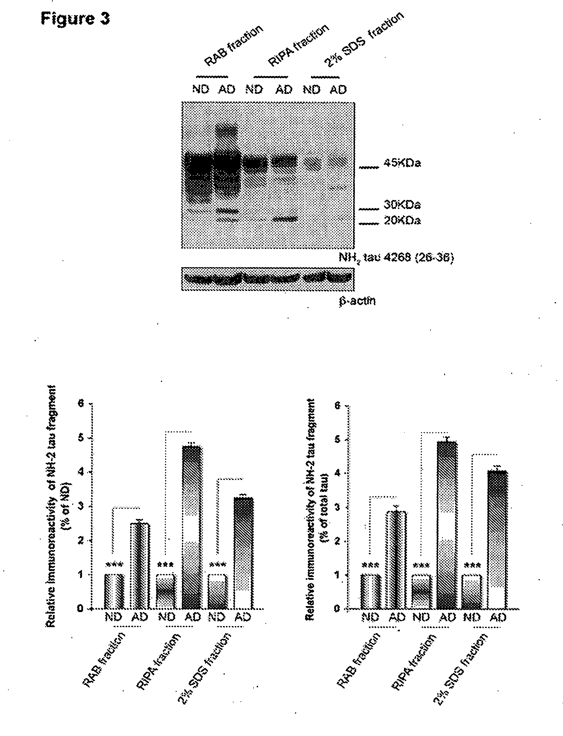

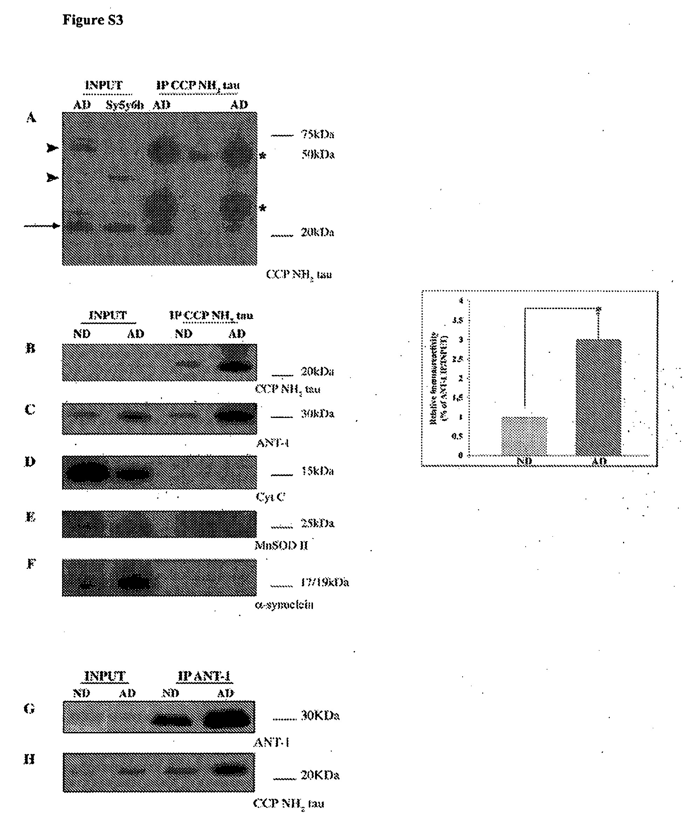

**Figure 3****.The solubility profile of the NH₂ 20-22 kDa tau fragment in AD brains**. The solubility profile of NH₂-derived 20-22 kDa tau fragment(s) from synaptosomal fraction of age-matched non-demented control (ND) and one representative AD (AD) subject has shown. The profile was generated using sequential buffers of increasing stringency: high salt, RIPA buffer, 2% SDS. An equal amount of the material from each extraction step (75 µg) was loaded for lanes and NH₂ 4268 tau antibody (1:600) was used for immunoblot analysis. Notice that, to reveal reaction with the NH₂ 20-22 kDa tau fragment, total protein amounts loaded for lane and antibody concentration used were increased. Densitometric analysis of intensity band- for the lower NH₂-derived 20-22 kDa tau fragment was shown beside and quantification was performed normalizing the sample towards correspondent β-acting amount, in according to [70]. The histograms show the ratio of NH₂-truncated tau fragment/β-actin (up) and NH₂-truncated tau fragment/ total full length tau (down) and the values were expressed as percentage to the respective control. Each data point is the mean ± SE (bars) of three independent experiments and statistically significant differences were calculated by paired t-Student's test (*p<0.05; ***p<0.001).

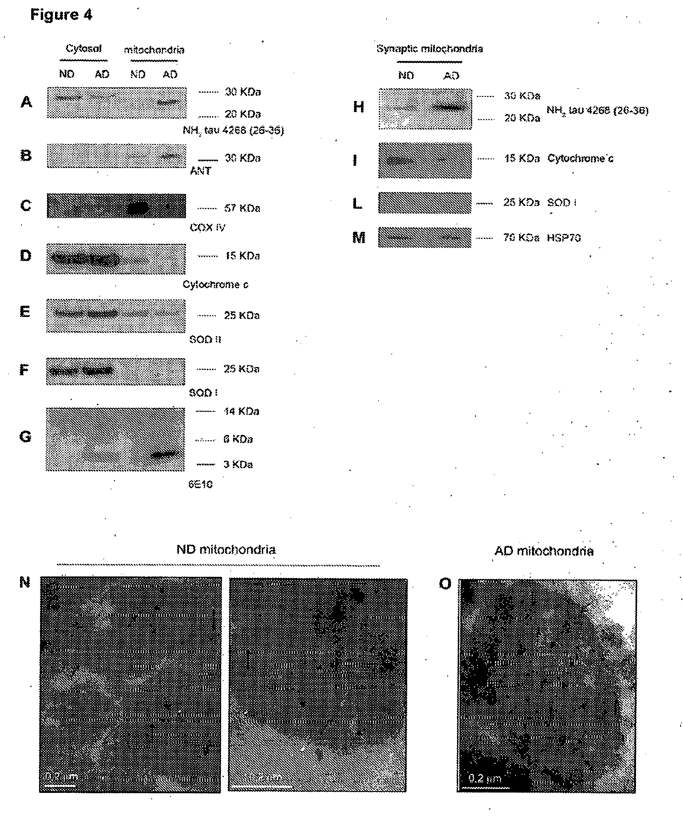

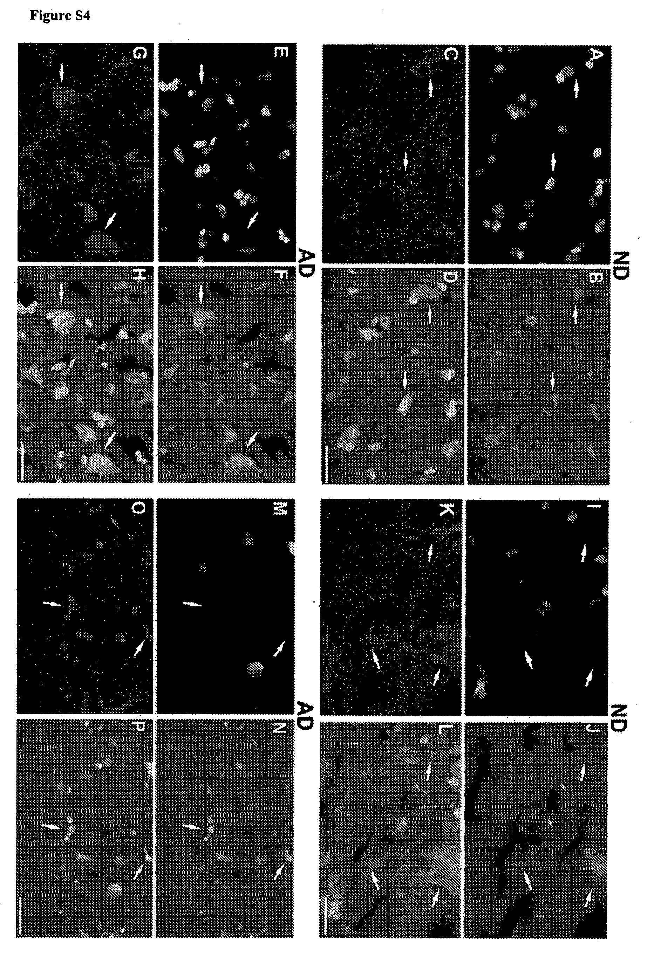

**Figure 4****. The pathological re-distribution of the NH₂-derived 20-22 kDa tau fragment in AD synaptic mitochondria is accompanied by organelle impairment.** A-B-C-D-E-F-G): The distribution of NH₂-derived 20-22 kDa tau fragment was compared in equal proteins amount of cytosolic and mitochondrial fractions (25µg) from non-demented control (ND) and one representative AD (AD) subject, by Western blotting with NH₂ 4268 tau (aa26-36) antibody (A). To ensure the enrichment of the sub-cellular fractionation, immunoblotting was performed with antibodies specific for mitochondrial cyt C (D) and MnSOD-II (E) -two proteins localized to intermembrane space and matrix-, for ANT (B) and COXIV(C)- other two proteins localized instead to inner membrane of organelle- and for cytosolic SOD-1 (F). The accumulation of Aβ peptide(s) in AD mitochondria was also checked with 6E10 antibody (G). H-I-L-M): Biochemical analysis of the synaptic mitochondrial localization of the lower NH₂-derived 20-22 kDa tau fragment has reported. Equal cytosolic and mitochondrial proteins amounts (25µg) from isolated synaptosomes of non-demented control (ND) and of one representative AD (AD) subject were assessed by Western blotting for NH₂ 4268 tau (aa26-36) (H), cyt C(I), SOD-I (L),and normalized to mitochondrial-specific HSP70 (M). N-O): Immunoelectron microscopy (TEM) of mitochondrial localization of the NH₂-derived 20-22 kDa tau fragment in human brain biopsy from AD subject (O) and non-demented age-matched control (N) has shown. Representative immunogold labeling (of n=3) with affinity-purified NH₂ 4268 tau (aa26-36) antibody (arrows) strongly decorated mitochondria in degenerating neurons of AD brain. Preadsorption of antibody with NH₂-26-44 synthetic peptide abolished labeling (not shown). Calibration bar 0.2µm.

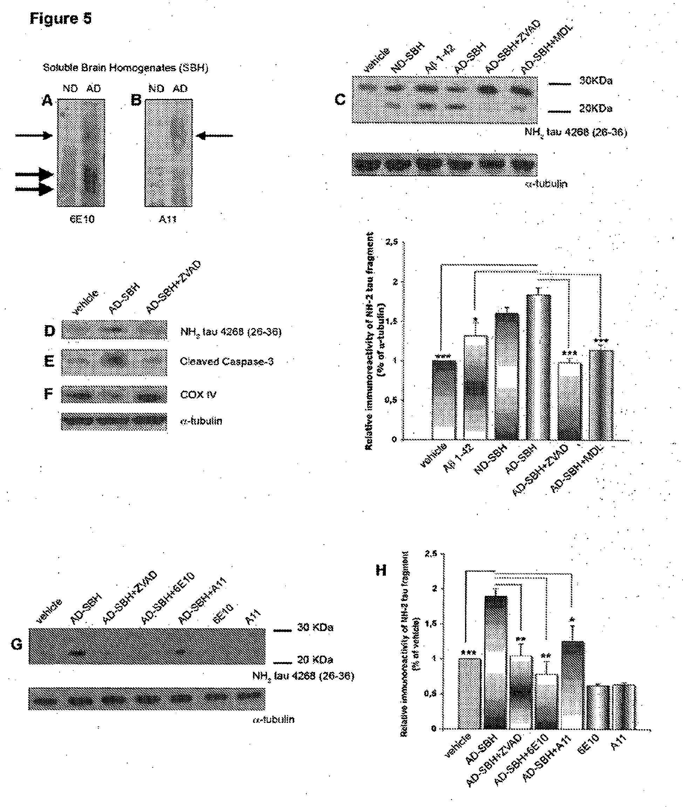

**Figure 5****. The soluble extracts from AD brain homogenates containing Aβ oligomers induce neurotoxicity and production of the NH₂-derived 20-22 kDa tau fragment through the caspase-(s) activation in human differentiated SY5Y. A-B)**: Biochemical characterization of native AJ3 oligomers enriched by crude soluble proteins extracts of AD and age-matched non-demented control brain prepared as reported by [57], which contained almost no Aß monomers since the ability of these low-molecular weight species to pass readily through the 10 kDa cutoff filters under the conditions used here. Samples extracted from two different AD brain specimens were pooled and retentates- obtained by 10 kDa cutoff filters (Vivaspin 4 VS0403)- were added to the cell culture at a final concentration of 1mg/ml. Equal amounts of proteins were separated by native (not-denaturating) 12% Tris-glycine PAGE separation with native mark unstained protein standard (Invitrogen LC0725). Unheated samples were applied on the gel in two different lanes and after transfer the membrane was cut in two equal parts and analyzed in parallel. Western blot with conformation-dependent A11 (B) and non-conformation-dependent 6E10(A) antibodies shows a shift toward larger oligomeric forms of Aβ peptides, demonstrating that AD soluble homogenates contained a mixture of trimers and higher molecular weight (HMW) Aβ oligomers. Proteins smear in HMW regions reacted with antibody specific for A11 and smear in ND sample was only faintly detectable (A, compare lane 1 with 2). C) dbAMPc/NGF-differentiated human SH-SY5Y human neuroblastoma cells were incubated for 12 h with 1mg/ml F12 oligomer-containing supernatants from AD brain crude homogenates (SBH) [60] -as well as from non-demented controls -or with synthetic 10µM Aβ 1-42 peptide, in the absence or in the presence of 100µM Z-VADfmk or 20µM MDL28170 - a pan-caspase(s) or calpain-I inhibitors respectively. Intact nuclei count (not shown) was also in parallel performed to monitor the neuronal cultures viability upon treatment (not shown). Western blots show the immunoreactivity of protein lysates probed with NH₂ 4268 tau (aa26-36) antibody. Identical protein amounts (25µg) were applied to each lane. As loading control, α-tubulin was used to allow direct comparison between the total proteins mass applied to different lanes. Panel shows the quantification of the ratio between the 20-22 NH₂ truncated tau fragment/α-tubulin expressed as percentage to the control. Each data point is the mean ± SE (bars) of five independent experiments and statistically significant differences were calculated by paired t-Student's test (*p<0.05; ***p<0.001). D-E-F) dbAMPc/NGF-differentiated human SH-SY5Y human neuroblastoma cells were incubated for 12 h with 1mg/ml F12 oligomer-containing supernatants from AD brain homogenates (SBH) [60], in the absence or in the presence of 100µM Z-VADfmk pan-caspase(s) inhibitor. Equal proteins amounts (25µg) were applied to each lane and α-tubulin was used as loading control. Western blots show the immunoreactivity of protein lysates probed with NH₂ 4268 tau (aa26-36) antibody (D), cleaved caspase-3 (E) and COXIV (F). G-H) dbAMPc/NGF-differentiated human SH-SY5Y human neuroblastoma cells were maintained for 12 h at 37°C with 1mg/ml F12 oligomer-containing supernatants from non-demented and AD homogenates poolled from two different brains (SBH), in the absence or in the presence or of 100µM Z-VADfmk, 2 µg/ml 6E10, 2µg/ml A11 antibody [60]. When present, inhibitor and antibodies were added to cultures 30 min before addition SBH. Equal proteins amounts (25µg) were applied to each lane and α-tubulin was used as loading control. Western blots show the immunoreactivity of protein lysates probed with NH₂ 4268 tau (aa26-36) antibody (G) and α-tubulin. Panel (H) shows the quantification of the ratio between the 20-22 NH₂ truncated tau fragment/α-tubulin expressed as percentage to the control. Each data point is the mean ± SE (bars) of three independent experiments and statistically significant differences were calculated by paired t-Student's test (*p<0.05; **p<0.01; ***p<0.001).

**Figure S2. Biochemical characterization of neuron-derived conditioned media from differentiated human SY5Y incubated with 10µM synthetic human Aβ 1-42 and oligomers-enriched AD soluble brain extracts (SBH).** A-B) Differentiated human SY5Y neurons were incubated for 12h with vehicle alone, with 10µM of soluble human Aβ 1-42 (61), with 1mg/ml AD-SBH [60]. Equal amounts of unheated and DTT-free conditioned melieu were loaded for lane and subjected to Western blotting analysis. After resolving on SDS-PAGE (4-12% Tris-glycine) and Ponceau staining to verify the homogeneous proteins loading, the filter was probed with 6E10 antibody (anti-Aβ4-9). Notice that the conditioned medium from Aβ peptide-exposed cultures contained a mixture of SDS-stable soluble dimers, trimer/tetramers while a faint immunoreactivity of oligomeric Aβ species was present in melieu from AD-SBH-treated neurons, which in contrast contained αAPPs and full length APP (A). However, several 6E10-immunoreactive, SDS-stable, Aβ aggregates-which theoretically corresponded to trimeric (14-kDa), hexameric (27 kDa), nonameric (40 kDa) and dodecameric (56 kDa) and HMW multiples of Aβ1-42 assemblies (50-75kDa)- were contextually detected in oligomeric-enriched AD-SBH preparation (S2B, left) suggesting that, upon 12h neurons treatment, such aggregates were *in vitro* largely membrane-bound or internalized (130,173). Finally when denaturated, by mild boiling or by low concentration of reducing agents -i.e.DTT-, the protein-smear in HMW region disappeared concomitant with a parallel increase in levels of examers and, to a lesser extent, of tetrameric/trimeric Aβ species (S2B, right). All together, these results suggest that (i) the 6E10-positive Aβ complexes are held together by SDS-resistant hydrogen bonds or S-S covalent bonds. In addition, as previously reported [75], the great resistance to trimers/tetramers to denaturating conditions confirmed that these Aβ aggregates are the primary Aβ assembly unit in vivo.

Arrows indicate respective migration positions of monomers (1-mer), dimers (2-mer), trimers (3-mer), tetramers (4-mer), hexamers (6-mer), nonamers (9-mer), dodecamers (12-mer) and sAPPa (secreted form of APP that has been cleaved by a-secretase). Asterisk indicates the protein-smear in high molecular region around 50-75 kDa.

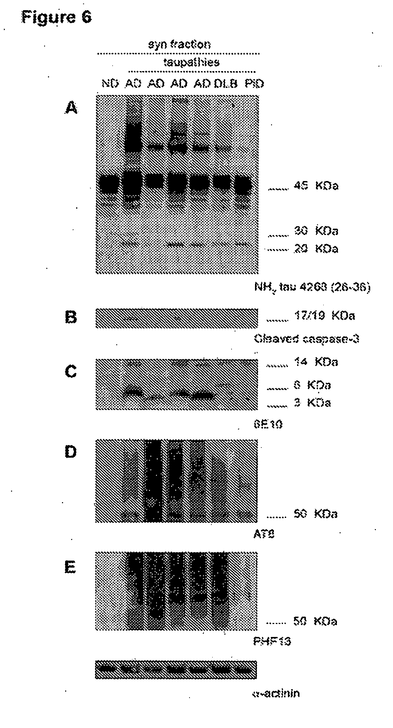

**Figure 6****. The NH₂ 20-22 kDa tau fragment is also present in other human caspase(s)-dependent not AD- tauopathies while its presence is linked to Aβ multimers and to pathology severity in AD patients.**

Western blotting analysis with NH2 4268 tau (aa26-36) (1:2000)(A), cleaved caspase-3 (B), 6E10 (C), AT8 (D) and PHF13 (E) antibodies of equal proteins amounts (20µg) of synaptosomal fractions from control (n=1), AD (n=4), DLB (n=1), PiD (n=1). The levels of the NH₂-derived 20-22 kDa tau fragment -except in young patient (A, lane 3)- are significantly increased in all diseased cases compared to control (A) and correlated with the presence of the active caspase-3 form (B). No or limited accumulation of 6E10-positive Aβ oligomeric assemblies was detected in not-AD tauopat (C, lanes 6-7), which indeed show a pathological tau hyperphosphorylation at comparable levels to other analyzed AD cases (D-E compare lanes 6-7 with lanes 2,3,4,5,6). Notice also that no Aβ aggregates and active caspase -3 were found in synaptosomes from young subject (lane 3), which suffered of mild/moderate AD and showed no significant level of the NH₂ 20-22 kDa tau fragment.

**Figure S3. NH₂-truncated tau specifically co-precipitates with ANT-1 in synaptic mitochondria of AD subjects**
A):Western blotting comparing synaptosomal proteins (500µg) from ND, AD (ND, AD) subjects immunoprecipitated (IP) and probed (WB) with CCP-NH₂ tau (NH₂ aa 26-36 of human tau) antiserum. Total proteins extracts (70 µg unbound) from STS-treated (6hSY5Y) neuronal human SY5Y and from AD synaptosomal proteins (INPUT) were also loaded for lane and included as positive internal control of electrophoretic mobility and immunoprecipitation efficiency. It is noteworthy that a NH₂-tau fragment around 20-22kDa (arrow), but not-full-length proteins running at 55-75kDa(arrowheads), was efficiently immunoprecipitated by CCP-NH₂ tau antiserum on synaptic-enriched fractions from AD. The asterisk indicate immunoglobulin light chains in immunoprecipitates and no significant cross-reactions with human intact tau isoforms was found in total, un-precipitated proteins.
B-F):In C), Western blotting analysis comparing synaptosomal proteins (500µg) from ND, AD (ND, AD) subjects immunoprecipitated (IP) with CCP-NH₂ tau (NH₂ aa26-36 of human tau) antiserum and probed (WB) with ANT-1 antibody (the least C-terminal 12 amino acids of human neuronal ANT-1). As positive internal control of electrophoretic mobility and immunoprecipitation enrichment, synaptosomal proteins from ND and AD cases (20-25µg INPUT) were also loaded for lane and included. In B), control experiments demonstrated the a NH₂-tau fragment around 20-22kDa was efficiently immunoprecipitated with CCP-NH₂ tau. The membranes were next stripped and reprobed with cyt C(fig1D),with MnSOD-II(fig.1E) and α-syn antibodies(fig.1F). Western blots shown were representative of at least three separate experiments
G-H): Synaptic-enriched mitochondrial fractions were purified from ND and AD subjects and proteins (1.5mg) were subjected to reciprocal immunoprecipitation (IP) with immunocapture mouse ANT-1 antibody (the least C-terminal 12 amino acids of human neuronal ANT-1). The immunoprecipitates were next probed with CCP-NH₂ tau (NH₂ aa26-36 of human tau) antiserum (H) and ANT-1 (least C-terminal 12 amino acids of human neuronal ANT-1)(G). Western blots shown were representative of at least three separate experiments

**Fig.S4****. The NH₂htau fragment colocalizes with ANT-1 in AD mitochondria**
A-H): Confocal images of double immunofluorescence with CCP-NH₂ tau (NH₂ aa26-36 of human tau, green channel) and mouse ANT-1 antibodies (the least C-terminal 12 amino acids of human neuronal ANT-1, red channel) of cerebral cortex from AD (F-G-H) and age-matched ND control case (B-C-D). Nuclear counterstaining with DAPI (cyan channel)(E-A, respectively). In the control, the mitochondrial ANT-1 immunoreactivity was largely detected throughout the entire tissue with only few intracellular granules distributed in perinuclear position. The CCP-NH₂ 4268 tau staining is low and diffuse in the cytoplasmatic domain and did not colocalize with ANT-1-immunopositive mitochondria (arrows). In the disease subject, the CCP-NH₂ tau and ANT-1 staining was more intense, highly colocalized and confined to intracellular aggregates (arrows) of neurons displaying a globose morphology. Immunofluorescence studies shown were representative of at least three separate experiments. Scale bar: N= 30 µm; R= 20 µm.
I-P) Confocal images of double immunofluorescence with CCP-NH₂ tau (NH₂ aa26-36 of human tau, green channel) and mouse ANT-1 antibodies (the least C-terminal 12 amino acids of human neuronal ANT-1, red channel) of terminals-enriched cerebral sections from Alzheimer disease (N-O-P) and age-matched non-disease control case (J-K-L). Nuclear counterstaining with DAPI (cyan channel)(I-M, respectively). In the disease subject, the CCP-NH₂ tau and ANT-1 staining was more intense and highly colocalized and confined to distal intracellular aggregates (arrows), away the DAPI-positive nuclei. In the control, the mitochondrial ANT-1 immunoreactivity was largely detected throughout the entire tissue without any obvious co-distribution with the CCP-NH₂ 4268 tau staining which, on the contrary, was only faintly appreciable (arrows). Immunofluorescence studies shown were representative of at least three separate experiments. Scale bar: N= 30 µm; R= 20 µm.

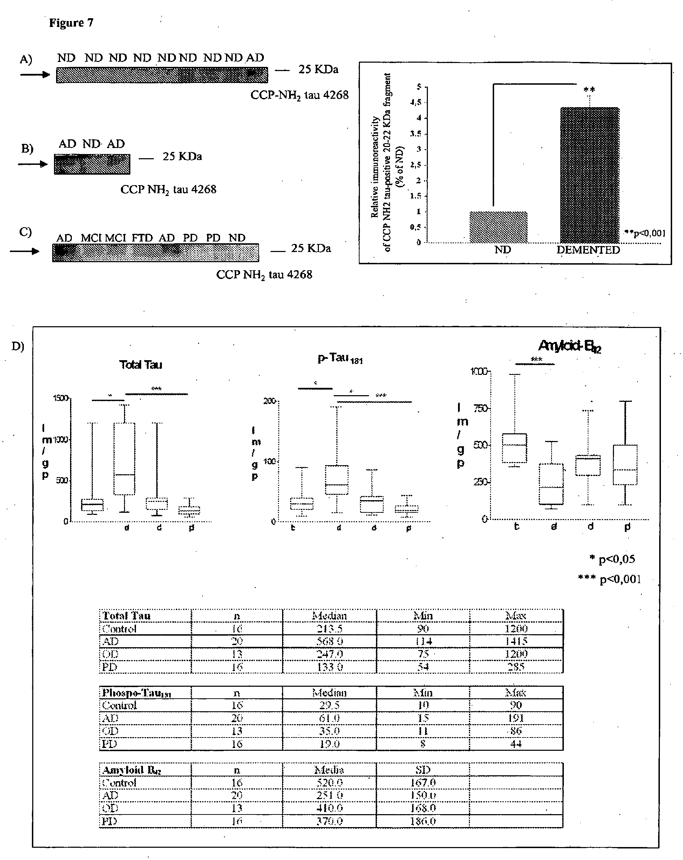

**Figure 7****. The 20-22kDa NH₂tau fragment is present in human CSF from AD patients as well as from patients affected by other tauopathies**
A-B-C:SDS-PAGE analysis of lyophilized 1 ml CSF from ND,FTD,PD, AD patients with CCP-NH₂ 4268 the purified form of antibody NH₂ 4268 antibody (aa26-36 of human tau). Notice that the CCP-NH₂ 4268 tau antibody-that only recognizes human tau protein truncated at D25 residue without any significant reactivity against the full length protein-detected the specific increase of the 20-22 kDa NH₂ tau fragment in CSF from early-middle (MCI) to late AD cases but not in age-matched,healthy controls. Densitometric data form all analyzed cases (not-demented n=30; demented n=60) showed a statistically significative increase of the 20-22kDa NH₂-derived tau fragment (around 4,5 fold, t-test p<0.001) in diseased subjects.
C: Comparison of CSF levels of total tau, phospho-tau(Thr 181) and Aβ1-42 detected by ELISA in patients affected by probable Alzheimer's disease (AD) (*n* = 20), patients affected by other dementias (OD) (n = 13), patients affected by Parkinosn's disease(PD) (n=16) and control patients affected by neurological diseases without cognitive impairment (CTRL) (n = 16). The samples from each patient were assayed *in vitro* in duplicate. Values, expressed as pg/mL, are represented as error bars with mean and C.I. 95%.. The Amyloid-β₁₋₄₂ levels were analyzed using parametric One-Way Analysis of Variance (ANOVA) followed by Bonferroni's test; h-Tau and phospo-tau₁₈₁ data were compared using Kruskal-Wallis test. (**p*< 0.01; **P < 0.001).

### MATERIALS AND METHODS

### Ethics statements

This study was performed in accordance with local ethics committee and with the principles contained in the Declaration of Helsinki as revised in 1996. All animals were handled and cared for in accordance with EEC guidelines (Directive 86/609/CEE). All ethics statement declarations will be enclosed in Ethics section.

### Brain material

Human brain material was provided via the rapid autopsy programme of the Netherlands Brain Bank (NBB), which provides post-mortem specimens from clinically well documented and neuropathologically confirmed cases (Table 1). All research involving them was conducted according to the ethical declaration of the Netherlands Brain Bank. Ethical approval and written informed consent from the donors or the next of kin was obtained in all cases. The work of the NBB abides by the ethical code of conduct approved by the ethics committee. Clinical diagnosis of probable AD was made according to the NINCDS-ADRDA criteria [50] and severity of dementia rated by the Global Deterioration Scale. All cases were neuropathologically confirmed, using conventional histopathological techniques, and diagnosis performed using the CERAD criteria [51]. Non-disease controls had no history or symptoms of neurological or psychiatric disorders and were clinically non-demented. All cases in control groups are gently provided by Prof. Bussani of UCO Anatomia e Istologia Patologica- Ospedale di Cattinara Trieste. All of non-demented human cases are retrieved from the autopsy specimen files of Ospedale di Cattinara-Trieste, under the approval of the local Ethical Committee. Subjects' details (age, post-mortem delay, autopsy number and brain weight approximately used for each single experiment) are shown in Table 1 and no statistically significant differences are present between non-demented controls and analyzed patients. To minimize the considerable regional differences in pathology, the authors selected to use hippocampus and/or frontal cortex- analyzed together -in all tested patients. CSF sample was taken by lumbar punctures following standard and it was also provided from the Netherlands Brain Bank (NBB).

### Animals

Young (3 months) and old (15 months) Wistar rats were from inbred colony of the Tg2576 (overexpressing human APP695 used as a AD model). Non-transgenic littermates were used as controls. All animals were handled and cared for in accordance with European Economic Community guidelines.

### Purification of cleavage-site CCP NH₂-Tau (aa 26-36 epitope) antibody rabbit (D25-(QGGYTMHQDQ)

Synthesis, HPLC purification, mass identification by mass spectral analysis of both the spanning and the cleavage-site NH₂ tau peptides and antibody purification procedures were performed by NEP laboratories (New England Peptide) 65 Zub Lane, Gardner,MA 01440 (project: P585398-M146310 NEP quote: cleavage-site specific antibody services). In detail, two peptides (the spanning and the cleavage-site NH₂ tau) was generated and each was conjugated to column. The NH₂ full-length tau (aa 26-36 epitope)-detecting antiserum [174] was run over the spanning peptide to adsorb out the full-length reactive material. The flow-through from this first column was run over the cleavage-site specific peptide column. The elution from this second column is the final cleavage-site specific purified antibody.

Sequence of the cleavage-site NH₂ tau peptide : AcQGGYTMHQDQEGDTDAGLKC-amide.

Sequence of the spanning peptide: Ac:YGLGDRKDQGGYTMHQC-amide. (SEQ ID No.2).

### Synaptosomal fractionation

Synaptosome-enriched subcellular fractions of brain were prepared according to [52]. These fractions, as described by others [53] are largely enriched in both pre- and postsynaptic proteins such as PSD95 and GluR2/3. In brief human brain was homogenized in 2 ml of homogenization buffer (320 mM sucrose/ 4 mM Hepes, pH7.4/ 1mM EGTA/ 0.4 mM PMSF/ plus proteases inhibitor cocktail (Sigma P8340) and phosphatase inhibitor cocktail (Sigma Aldrich, Oakville, Ontario, Canada P5726/P2850) with 15 strokes of a glass Dounce tissue grinder (Wheaton). The homogenate was centrifuged at 1000Xg for 10 min. The supernatant was collected and centrifuged at 12000Xg for 15 min, and the second pellet was resuspendend in 2 ml of homogenzation buffer and centrifuged at 13000xg for 15 min. The resulting pellet was resuspended in 0.3 ml of homogenization buffer.

### Isolation of mitochondria

The mitochondrial extraction was performed using mitochondrial extraction kit (Qiagen 37612) according to the manufacturer's instructions. In brief human brain was homogenized in 0.5 ml of homogenization buffer using a tissueruptor rotor-stator and incubated on an end-over-end shaker for 10 min at 4C. The homogenate was centrifuged at 1000xg for 10 min at 4°C. The resulting pellet was resuspended in disruption buffer, repeatedly passed through a narrow-gauge needle (26 or 21 gauge), and re-centrifuged at 1000 X g for 10 min; the pellet contains nuclei, cell debris, and unbroken cells. The supernatant, which contains mitochondria, was re-centrifuged at 6000 X g for 10 min. The mitochondria pellet was resuspended in Mitochondria Purification Buffer and slowly the Disruption Buffer was pipetted under the Purification Buffer. The mitochondrial suspension was pipetted on top of layers and centrifuged at 14000 X g for 15 min at 4°C. The Mitochondria pellet was resuspended in mitochondria storage buffer.

### Preparation of total protein extracts from human brains

For analysis of total protein brain extracts [54], blocks (about 0.1 g) of frozen tissue were dissected into 5 vol. of lysis buffer (20mM Tris pH 7.4 plus protease inhibitors), homogenized on ice and centrifuged at 13,000 X g for 15 min. The supernatant was quantified and analyzed by SDS-PAGE and Western blot.

### Isolation of tau from Biopsy Human Brain Samples

Tau was isolated from Biopsy Human Brain Samples according to [55]. In brief the brain samples were homogenized in ice-cold reassembly buffer (0.1 M MES, 0.5 mM MgSO4, 1 mM EGTA, 2mM dithiothreitol pH 6.8, 0.75 M NaCl plus proteases inhibitor cocktail (Sigma P8340) and phosphatase inhibitor cocktail (Sigma Aldrich, Oakville, Ontario, Canada P5726/P2850) and centrifuged at 50000Xg for 30 min at 4°C. The supernatant was boiled for 10 min and re-centrifuged at 50000Xg for 30 min. The supernantant was then collected. Proteins concentration was determined using the Bio-Rad RC DC protein assay kit.

### Tau solubility

The solubility of tau in human brain samples was studied according to [56] with modifications. In brief the brain samples was homogenized using buffers of increasing stringency: (1) buffer A (high salt, 750 mM NaCl, 50 mM Tris buffer, pH 7.2), (2) RIPA buffer (50 mM Tris/HCl, pH 7.5, 150 mM NaCl, 1 mM EDTA, 1% NP-40, 0.5% deoxycholate, and 0.1% SDS, pH 8.0), (3) 2% SDS. At each step the samples were briefly sonicated, centrifuged at 45,000 X g for 30 min at 4°C, and supernatants were collected.

### Preparation of soluble brain extracts enriched soluble Aβ assemblies

Brain extracts was performed according to [57]. In brief the brain samples were homogenized in 20 volumes of F12 plus proteases inhibitor cocktail (Sigma P8340) and phosphatase inhibitor cocktail (Sigma Aldrich, Oakville, Ontario, Canada P5726/P2850) using a TissueRoptor (GLH 220, Omni International) and was centrifuged at 100000 X g for 1 h. The supernatant was collected and the pellet was rehomogenized in 10 volumes of F12 plus protease and phosphatase inhibitor cocktail and was recentrifuged. The combined supernatants were collected and were then concentrated by using a Centricon-10 concentrator (Vivaspin 4 VS0403 10,000 MWCO PES).

### TEM (Transmission Electron Microscopy)

Samples were cut into small pieces (1-2 mm³), fixed with 4% paraformaldehyde plus 0.5% glutaraldehyde in PBS, pH 7.4, for 24 h at 4°C, washed in PBS and then infiltraded with 2.3 M sucrose in PBS overnight at 4°C, frozen in liquid nitrogen and cryosectioned following the method by Tokuyasu (1973) [58]. Ultrathin cryosections, obtained by using a Leica Ultracut UCT device (Leica Microsystem, wien, Austria), were collected using sucrose and methylcellulose and incubated overnight at 4°C with the specific primary affinity-purified NH₂ 4268 tau antibody and then with anti-human IgG 5 nm gold conjugated (Sigma-Aldrich, Milan, Italy). Finally, ultrathin cryosections were stained with 2% methylcellulose and 0.4% uranyl acetate solution. Samples were examined with a Philips 208 transmission electron microscope (FEI Company, Eindhoven, The Netherlands).

### Cell culture and treatments

SH-SY5Y human neuroblastoma cells (American Type culture Collection, Rockville, MD, U.S.A.) were grown in DMEM/F12 medium (Invitrogen, Gibco BRL 31331-028) supplemented with 10% fetal calf serum (Invitrogen, Gibco BRL 10108-157), 100 U/ml of penicillin and 100 µg/ml of streptomycin (Invitrogen, Gibco BRL 15140-122) and maintained at 37°C in a saturated humidity atmosphere containing 95% air and 5% CO₂. The medium was changed every two days. Naive cells at 20% confluency were differentiated by incubation for 5-6 days in DMEM/F12 medium containing N2 (Invitrogen, Gibco BRL 17502-048), dbcAMP 1 mM (Sigma-Aldrich, Oakville, Ontario, Canada-D-0627) and NGF 100 ng/ml. For all treatments, the media with additives were changed after 1 and 3 days. Apoptosis was induced by addition of Staurosporine (Sigma-Aldrich, Oakville, Ontario, Canada S-4400) (0.5 µM), prepared as a stock solution in dimethylsulfoxide (DMSO), at 6 hours.

Hippocampal neurons were prepared from embryonic day 17-18 (E17/E18) embryos from timed pregnant Wistar rats (Charles River), as previously reported by [59]. In detail, the hippocampus was dissected out in Hanks' balanced salt solution buffered with HEPES and dissociated via trypsin/EDTA treatment. Cells were plated at 1 x 10⁶ cells on 3.5cm dishes pre-coated with poly-DL-lysine. After 2 days of culturing in neurobasal medium with B-27 supplement and glutamax, cytosine arabinofuranoside was added to reduce glial proliferation. Half of the medium was changed every 3-4 days. Cultures were pre-treated with ZVAD-fmk (100µM), MDL28170 (20µM) at the concentration reported for 1h and then exposed to oligomeric-containing brain homogenates (1mg/ml)+inhibitor for additional time, as previously reported by [60] (see figure legend). For Aβ 1-42 treatment of hippocampal neurons [61], a 1mM stock solution was obtained by dissolving Aβ1-42 in DMSO:H₂O (1:1) and similar amounts of DMSO were incubated in the control sample medium. All experiments were carried out by incubating cultures with 10µM Aβ 1-42 for 12h. Synthetic Aβ oligomers were prepared as described by [62] and used at final concentration of 2.5 mM.

### Assessment of neuronal viability

Cell viability was quantified by counting the number of intact nuclei, after lysis in detergent-containing solution [63].

### Protein cellular lysates preparation.

Total proteins were extracted by scraping the cells in an SDS-reducing sample buffer or lysis in ice-cold RIPA buffer (50 mM Tris-HCl pH 8, 150 mM NaCl, 1% Triton, 2 mM EDTA, 0,1% SDS plus proteases inhibitor cocktail (Sigma P8340) and phosphatase inhibitor cocktail (Sigma Aldrich, Oakville, Ontario, Canada P5726/P2850) and centrifuged at 4 °C for 15 min at 1000 X g. The supernantant was then collected and boiled for 5 min. Proteins concentration was determined using the Bio-Rad RC DC protein assay kit.

### Western blot analysis

Equal amounts of protein were subjected to SDS-PAGE 7.5-15% linear gradient ot Tris/Tricine 4-12%(NuPage, Invitrogen). After electroblotting onto a nitrocellulose membrane (Hybond-C Amersham Biosciences, Piscataway, NJ) the filters were blocked in PBS containing 5% non-fat dried milk for 1h at room temperature or overnight at 4°C. Proteins were visualized using appropriate primary antibodies. All primary antibodies were diluted in 0.5% (w/v) nonfat dry milk, and incubated with the nitrocellulose blot overnight at 4°C. Incubation with secondary peroxidase coupled anti-mouse, anti-rabbit or anti-goat antibodies was performed by using the ECL system (Amersham, Arlington Heights, IL, U.S.A.). Since different antibodies have different affinities for tau [55], the amount of total protein loaded and the concentration of the primary antibody used is specified in figure legend or in material and methods. Quantity One software, associated with the versaDoc imaging system (Bio-Rad) was used to quantify resultant bands of interest. Native gels were run in the absence of SDS and reducing agents and without heating samples.

Probed filters were stripped by using stripping buffer (100mM 2-Mercaptoethanol, 2% SDS, 62.5 mM Tris-HCl pH 6.7) at 50°C for 30 minutes with occasional agitation.

The following antibodies were used:
NH₂-CCP tau antibody rabbit (D25-(QGGYTMHQDQ) epitopes-phosphorylation independent state) ( 1:500); NH₂-Tau 4268 (aa 26-36 epitopes ) rabbit Sigma Aldrich, Oakville, Ontario, Canada (1:1000); HT7 (aa159-163 epitopes phosphorylation independent) mouse 90222 Immunogenetics, Autogen Bioclear, UK(1:1000); AT100 (Thr 212,Ser 214 epitopes-phoshorylation dependent state) mouse 90209 Immunogenetics, Autogen Bioclear, UK(1:1000); AT8 (Ser-202, Thr-205 epitopes-phoshorylation dependent state mouse) Innogenetics (1:1000); PHF13 (Ser396 epitopes-phoshorylation dependent state) mouse Innogenetics (1:1000); AT180 (Thr-231 epitopes-phoshorylation dependent state) mouse 90337 Immunogenetics, Autogen Bioclear, UK(1:1000); DC39N1 ( aa 45-73 epitopes, first N-terminal Insert ) mouse T8451 Sigma Aldrich, Oakville, Ontario, Canada (1:500); Tau 13 (aa1-13) mouse sc-21796 Santa-Cruz Biotechnology, USA(1:1000); tau 21 (aa21-36) rabbit AHB0371 Biosource International (USA) (1:250); Alz50 mouse was kindly provided by Dr. V. Lee (Department of Pathology and Laboratory Medicine, University of Pennsylvania School of Medicine, Philadelphia, PA); anti-amyloid β protein 4G8 (aa 17-24) mouse MAB1561 Chemicon (1:1000); anti-amyloid β protein 6E10 (aa 4-9) mouse MAB1560 Chemicon (1:1000); A11 anti amyloid beta, micellar (oligomeric) rabbit AB9234 Chemicon, Temecula, CA (1:1000); anti-Alzheimer precursor protein 22C11 (aa66-81 of N-terminus) APP-MAB348 Chemicon Temecula-CA (1:1000); Cleaved (Asp175) caspase-3 antibody rabbit 9661 Cell Signaling Technology, Beverly, MA (1:1000); anti α-synuclein (Ser 129) rabbit Imgenex, (1:1000); α-synuclein antibody mouse 610787 BD Transduction Laboratories (1:1000); ANT (anti-adenine nucleotide translocase ) mouse AP 1034 Calbiochem San Diego, CA ( 1:200); Phospho-cofilin (Ser3) antibody rabbit 3311 Cell Signaling Technology, Beverly, MA (1:1000); Cytocrome c (136F3) rabbit 4280 Cell Signaling Technology, Beverly, MA (1:200); Fyn antibody rabbit 4023 Cell Signaling Technology, Beverly, MA (1:1000); GAPDH (clone GAPDH-71.1) mouse G8795 Sigma Aldrich, Oakville, Ontario, Canada (1:5000); PSD95 antibody rabbit 2507 Cell Signaling Technology, Beverly, MA (1:1000); SNAP 25 ( synaptosome associated protein of 25kDa, C-terminal-specific) rabbit SL3730 Biomol International, LP (1:1000); Synapsin I antibody rabbit AB1543P Millipore Corporation, Billerica, MA (1:1000); Synaptophysin antibody mouse VAM-SV011 Stressgen Biotechnologies, Victoria, BC Canada ( 1:1000); PS1 antibody mouse SIG-39190 Covance, Princeton, NJ (1:1000); SOD II (MnSOD, mitochondrial superoxide dismutase) rabbit SOD-110D Stressgen Biotechnologies, Victoria, BC Canada ( 1:5000); SODI (cn/ZnSOD, cytosolic superoxide dismutase) rabbit SOD-100D Stressgen Biotechnologies, Victoria, BC Canada (1:5000); mtHSP70 (Anti-Mitochondrial Heat Shock Protein 70) mouse MA3-028 Affinity Bioreagents, Rockford, IL (1:500); anti-COXIV subunit I mouse A6403 Molecular Probes (1:500); anti-α-actinin-2 rabbit sc130928 SantaCruz Biotechnology,USA (1:5000); α-tubulin mouse T-5168 Sigma-Aldrich (1:1000); β-acting mouse S3062 Sigma-Aldrich (1:5000); ANT (H-188) rabbit sc-11433 Santa Cruz (1:500); ANT (5F51BB5AG7) mouse AP1034 Calbiochem (1:200); immunocapture ANT antibody (clone 5F51BB5AG7) mouse Mitoscience MSA01.

### Immunoprecipitation

The samples (600 µg total SY5Y protein extracts and human synaptosomal fractionation) were lysed using immunoprecipitation (IP) buffer (50mM Tris, pH 8.0, 150mM NaCl, 10% glycerol, 0,5 mM EDTA, 0,5 mM EGTA, 50mM NaF, 1mM NaOV₄, 1% NP40) supplemented with proteases inhibitor cocktail (Sigma P8340) and phosphatase inhibitor cocktail (Sigma Aldrich, Oakville, Ontario, Canada P5726/P2850). Lysates were then centrifuged at 4°C for 10 min at 3000 rpm and the supernatants were precleared using 5µl of protein G ( Invitrogen 100.03) for 1h at 4°C. The pre-cleared protein extracts was immunoprecipitated by cross-linking the Ig's antibodies to Protein G on bead surface-according to the manufacturer's instructions- with 4µg of CCP- NH₂ 4268tau for 10µl of protein G and eluted with Laemmli buffer 1X plus DTT (Invitrogen). The immunocomplexes were next analyzed by immunoblotting with ANT antibody (1:200) from Calbiochem (clone 5F51BB5AG7).

Reciprocal immunoprecipitation was performed with 50µl of mouse immunocapture ANT antibody (clone 5F51BB5AG7) from Mitoscience (MSA01) for 1.5mg of mitochondrial protein extract, according to the manufacturer's instructions. The immunocomplexes were next analyzed by immunoblotting with CCP- NH₂ 4268tau (1:600).

### Immunofluorescence and confocal microscopy

Tissue slabs derived from AD and ND cases were placed in a solution of 4% paraformaldehyde in 0.1 M phosphate-buffered saline (PBS, pH 7.2) at +4° for three days. After three washes in PBS 10 min each, slabs were placed in a solution of 30% (w/v) sucrose in PBS at +4° until they sank. The cryoprotected slabs were cut on a freezing microtome into 40 mµ thick sections and collected in a culture well for immunofluorescence procedures. For double immunofluorescence procedures the following combinations of primary antibodies were used: anti-ANT-1 (clone 5F51BB5AG7) mouse 1:1000; anti- CCP NH₂ tau rabbit 1:200. Before primary antibodies incubations, sections were heated at 90° for 1 h to unmask epitope and, after extensive washing, were first incubated 48 h at +4 degrees with primary antibodies, in 0.3% Triton X-100 in PBS. After three times washing in PBS sections were incubated with a mix solution of donkey anti-rabbit Alexa 488 (1:100) and donkey anti-mouse rhodamine-conjugated (1:100) secondary antibodies (Invitrogen) for 2 h at room temperature. The sections were next washed three times in PBS and then incubated with the nuclear marker, DNA-fluorochrome 4',6-diamidino-2-phenylindole (DAPI, 1:1000, Sigma-Aldrich) for 10 minutes, for nuclei visualization, followed by a further rinse. Sections were mounted on slides, air dried and coverslipped using gel mount (Biomeda Corp., Foster City, CA, USA). Control sections consisted of tissue from diseased cases which were incubated in the same conditions described above but with the exception of the primary antibody, and a preadsorption control consisting of a 100 fold amount of synthetic NH₂ 26-44 tau incubated with relative antibody in PBS containing 0.25% Triton X-110, 1% goat serum overnight at room temperature. Slides were examined under a confocal laser scanning microscope (Leica SP5, Leica Microsystems, Wetzlar, Germany) equipped with four laser lines: violet diode emitting at 405 nm, argon emitting at 488 nm, helium/neon emitting at 543 nm and helium/neon emitting at 633 nm. Confocal acquisition setting was identical between ND and AD cases. For production of figures, brightness and contrast of images were adjusted by taking care to leave a tissue fluorescence background for visual appreciation of the lowest flouorescence intensity features and to help comparison between the different experimental groups. Final figures were assembled by using Adobe Photoshop 6 and Adobe Illustrator 10.

### Statistical analysis

Experiments were carried out in triplicates and repeated at least three times. Data were expressed as means ± S.D. (n=4). Difference between control and treated groups were analyzed with SPSS software by one-way analysis of variance (ANOVA) for repeated measures followed by post-hoc Bonferroni test for multiple comparisons, except where otherwise stated. Statistical differences were determined at p<0.05 (*p<0.05; ***p<0.001) and are indicted in the figure legends. Experimental plots were obtained using Grafit (Erithacus software). For Western blot analysis and immunofluorescence studies shown were representative of at least three separate experiments.

In detail, the Amyloid-β₁₋₄₂ levels in CSF were analyzed using parametric One-was Analysis of Variance (ANOVA) followed by Bonferroni's test; h-Tau and phospo-tau₁₈₁ levels in CSF were compared using Kruskal-Wallis test; the NH₂-truncated tau fragment levels in CSF was analyzed using t-test Student(** p<0.001).

### EXPERIMENTAL PROCEDURES OF CSF ANALYSIS

### Patients and methods.

Study subjects were recruited from the Neurologic Clinic of University Hospital Tor Vergata during 2009-2010 years, with approval of Ethics Committee. Clinical diagnosis of probable AD was made according to the NINCDS-ADRDA criteria (Varma et al., 1999). The cohort consists of 66 CSF from 20 probable Alzheimer's disease (AD); 13 patients affected by other dementia, included 3 Frontotemporal and 4 vascular dementia, a case of Wernike-Korsakov syndrome and a spongiform encephalopathy; 16 Parkinson's disease (PD) and 16 age matched control subjects affected by other neurological disease (Control). All patients underwent a complete clinical investigation, including medical history, neurological examination, mini mental state examination (MMSE), a complete blood screening (including routine exams, thyroid hormones, level of B12), neuropsychological examination, a complete neuropsychiatric evaluation and magnetic resonance imaging (1,5 T MRI). Exclusion criteria were the following: 1) patients with isolated deficits and /or unmodified MMSE (≥ 25/30) on revisit (6,12,18 months follow-up), patients with clinically manifest acute stroke in the last 6 months showing an Hachinsky scale > 4, and a radiological evidence of sub-cortical lesions, patients with brain trauma or tumors.

### CSF withdrawal

CSF samples were collected as previously described (Sancesario et al., 2009). Six-eight ml was taken in polypropylene tube without preservative, gently mixed and immediately carried to the clinical lab. One sample was used for routine chemical analysis and microscopic observation; the other one was centrifuged at 2000 rpm at 4° for 10 min and frozen at - 80°. Blood samples were taken at the same time to evaluate blood brain albumin ratio and blood brain barrier integrity.

### CSF determination of amyloid-β₁₋₄₂, total and phospho tau₁₈₁

The levels of specific biomarkers in the CSF were determined using commercially available sandwich enzyme-linked immunosorbent assays (Innotest β-amyloid 1-42, Innotest h-Tau Ag, Phospo-tau₁₈₁, Innogenetics, Ghent, Belgium) (Sancesario G.M., 2009). Briefly, 25 or 75 µl of the CSF from each patient were dispensed into corresponding 96-well ELISA plates, coated either with the monoclonal antibody 21F12 for Aβ1-42, AT120 for h-Tau and HT7 for phospho-tau and incubated with the respective biotinylated antibody 3D6, HT7 and BT2, or AT270. Finally, bound antibodies were detected by a peroxidase-labeled streptavidin, after addition of a substrate solution. The reaction was stopped by sulphuric acid, and the absorbance of the reaction product was read at 450 nm. The biomarkers concentrations in the samples were calculated based on the Aβ1-42, h-Tau and phospo-tau standard sigmoid curve equation.

### RESULTS

### The biochemical localization of a NH₂ human tau fragment in AD human synapse(s)

The authors examined the *in vivo* biochemical expression and subcellular distribution of the NH₂-derived 20-22 kDa tau fragment in synaptic-enriched fractions, obtained upon biochemical fractionation of synaptosomes [52] from human tissues. To this aim, cryopreserved autoptic samples of human brain areas -obtained from clinically and histopathologically diagnosed AD patients and from aged non-demented control- have been analyzed. Case demographics (age, post-mortem delay, brain area and relative weight) and neuropathological diagnosis are shown in Table 1 and no statistically significant differences were present between non-demented controls and AD patients.

**Table 1. Cases of autopsy-confirmed AD and healthy control subjects used in this study.**

| Cases analyzed for pathology-defined group. Alzheimer's Disease: AD; Pick's disease: PiD; DLB: Dementia with Lewy Bodies; ND non-demented healthy control subject. The brain weight indicates approximately the tissue amount used for each single experiment. | | | | | | |
|---|---|---|---|---|---|---|
| **Postmortem cases** | **No. of cases** | **Brank Stage** | **Age(range)(years)** | **Postmortem interval (min)** | **Region used** | **Brain weight(mg)** |
| ND | 5 | 0-I | 50-70 | 540 | hippocampus, cortex | 250-300 |
| AD | S | V-VI | 65-90 | 225-310 | hippocampus, cortex | 200-400 |
| Young AD | 1 | II-III | 49 | 250 | luppocampus, cortex | 200-300 |
| DLB | 1 | | 84 | 365 | temporal cortex | 200 |
| PiD | 1 | | 72 | 235 | hippocampus | 300 |

The authors selected the hippocampus and/or cerebral cortex in all tested patients since, as documented by metabolic/molecular neuroimaging data and clinical tests, damage in these selective brain areas in AD is related to some of its amnesic and behavioral abnormalities [64-66]. In addition, it has been proposed that loss of synapses in such brain areas represents the most severe neurobiological or cognitive impairment in AD [1,2,67].

The purity of the present fractionated preparation was tested to ascertain if it was really enriched in synaptic terminals and was free of contaminations from neuronal perikarya. To this aim the authors analyzed by Western blotting the expression level of specific presynaptic, postsynaptic and cell body proteins in total brain and synaptosomes extracts from human controls. As shown in fig.1, the presynaptic protein synapthophisin (fig.1A) and the postsynaptic proteins PSD95 (fig.1B) were highly enriched in the authors' synaptosomal preparation. On the contrary GAPDH, a protein present in the cell body (fig.1C) was found more abundant in total brain extract than in synaptosomes preparation. In addition, no difference in the intensity level of cytoskeleton elements-such as α-actinin-2 (fig.1D) and β-actin (not shown)- was detected in the authors' preparation between total and synaptosomes-enriched protein extracts, in agreement with [68-70].

For their following experiments, the authors decided to make use of a phophoindependent *ad*-*hoc* devised NH₂ tau antibody from here on called NH₂ 4268 tau. This rabbit-derived antiserum recognizes the 26-36 epitopes located on the extreme NH₂-domain of human tau protein -in a similar way of CCP-NH₂ tau antibody [35,46]- but it is not full-length tau pre-absorpted (for antibody specificity see fig. S1). The authors compared, by Western blotting with this specific NH₂ tau antibody [35,46], the expression levels of NH₂-derived 20-22 kDa tau fragment in total as well as in synapses-enriched protein extracts from diseased and control human brains. As shown in fig.1E-F, in contrast to total human brain extracts in which only full-length tau isoforms were detectable (fig.1E), the authors found a preferential distribution of the 20-22 kDa NH₂ tau fragment [46] in synaptic compartments (fig.1F) and- more interestingly- a significant higher expression level of this truncated tau form in AD synaptosomes compared to age-matched non-demented controls. It is noteworthy that NH₂ 4268 tau antibody recognizes by Western blotting an immunoreactive doublet ranging between 20 and 30 kDa in synaptosomes from AD and -with a minor intensity- from non-demented cases. Moreover, as shown in fig. 1G the fast migrating resolved band is more abundant than the slower in AD (n=15) but not in control (n=10) (around 9-10 fold higher). It is not clear whether the doublet arises from cleavage at the two nearby caspase(s) sites in the NH₂ -tau domain- i.e. caspase-3 and -6 [35,71]- or represents a post-translational modification of a single NH₂ tau fragment -i.e ubiquitination/phosphorylation. Alternatively, since the larger of the two proteolytic fragments had a stronger signal on Western blot in correlation to faint immunoreactivity of the smaller, it is possible that the latter fragment represents a proteolytic processing intermediate. In addition, as shown in fig.IF, the synapse(s)-localized NH₂-derived truncated tau form exhibited similar electrophoretic mobility and immunoreactivity with NH₂ 4268 tau antibody to the *in vitro* fragment from differentiated human SH-SY5Y cells [46] undergoing caspase(s)-dependent apoptosis following 6h staurosporine treatment [72,73]. A similar pattern of synaptic localization was detected for active caspase-3 -in agreement with Louneva et al., 2008 [70], as shown by probing the fractionated human extracts with antisera directed against the respective cleaved forms of enzymes (fig.1H-I). Taken together, these data clearly demonstrate that both NH₂-derived tau species were faintly observed in human extracts from total brain homogentates, when the filter was exposed for the time necessary to detected full-length 55-75kDa tau protein isoforms, because of their preferential distribution in synaptic compartments. Based on these findings, the authors decided to focus their attention in subsequent experiments mainly on the lower NH₂ tau fragment of 20-22 kDa molecular weight (comprising the sequence from aa 26 to amino acid 230 or a fragment thereof).

Since soluble pre-fibrillar Aβ1-42 oligomers induce neurotoxicity in rat organotypic hippocampal slices by triggering the caspase-3 activation and tau cleavage [74], the authors tested if the presence of the 20-22 kDa NH₂ tau fragment in AD synaptic compartments was associated to neurotoxic Aβ species. Therefore the authors reacted their fractionated synaptic-enriched extracts from diseased and control brains with 6E10, an antibody directed at the extracellular portion of the Aβ domain within amyloid precursor protein AβPP (residues 4-9 of human Aβ). As shown in fig.1F-L, the authors found that in AD synaptic compartments-but not in age-matched human control-the high synaptic level of 20-22 kDa NH₂ tau fragment (fig.1F) correlated with the presence of 4kDa Aβ monomer and with the SDS-resistant high molecular weight (HMW) oligomers (fig.1L), including 12mer 56kDa (Aβ56*), which has been previously reported to contribute to AD cognitive deficits by impairing the memory independently of plaques or neuronal loss [75]. Finally, the NH₂ 20-22 kDa tau fragment was also selectively enriched in synaptosomes of 3 month-old Tg2576 mice (also termed APP₆₉₅SWE), a transgenic AD model which shows an Aβ-dependent synaptic deficits and impaired long-term potentiation (LTP) [13,75], in correlation with a significant up-regulation of active caspase-3 (not shown).

These results suggest that (i) the NH₂-derived 20-22 kDa tau fragment is present at higher level in AD brain and is localized predominantly in synapse(s)and (ii) its *in vivo* distribution in synaptic compartments correlates with the presence of neurotoxic Aβ peptide species and with the active form of caspase-3.

### The high levels of the NH₂ 20-22 kDa human tau fragment in human synaptosomes are correlated with AD synaptic alterations

In order to assess if the presence of the NH₂ human tau fragment is related to synaptic alterations, the authors compared its immunoreactivity intensity to the expression level/distribution of specific pre- and post- synaptic markers in synaptosomes from AD and non-demented human brains.

As shown in fig.2, the synaptic distribution of the NH₂-derived 20-22 kDa tau fragment (fig.2A) was correlated to a significant decrease of expression of α-synuclein (fig.2B), synaptosome-associated protein of 25kDa (SNAP-25) (fig.2C), synapsin I (fig.2D). These three proteins are mainly localized in pre-synaptic nerve terminals, are involved in vesicle trafficking and neurotransmitter release and their neuronal immunoreactivity declines at more advanced AD neuropathological stages [17,22,76-80]. On the contrary, as previously reported by others [81], the authors contextually found an increase of immunoreactivity for PSD95 (fig.2F) -a postsynaptic density protein, which is involved in NMDA and AMPA receptor signalling- probably as result of a compensatory dendritic outgrowth. Moreover the expression level of synaptophysin (fig.2E), another presynaptic protein that is involved in vesicle exocytosis, was unchanged.

The local distribution of the NH₂ 20-22 kDa tau fragment in human AD synapses was linked to dendritic spine alterations, as documented by the increase of Ser3-phosphorylated cofilin (fig.2G), an actin-binding protein which is found in Hirano bodies in the brain of AD patients [82] and involved in Aβ-mediated neuritic dystrophy [83,84]. Finally, in direct correlation with the distribution of the NH₂ 20-22 kDa tau fragment(s) in humanAD synaptosomes, the authors detected an increase of the synaptic levels of p59Fyn (fig.2H). This Src-family tyrosine kinase is localized to synaptic sites [85], since it is involved in learning and memory processes [86], and its alterations are found to be associated to synaptic AD decline, in tight colocalization with paired-helical-filaments (PHFs)-tau [87,88].

Next the authors attempted to explore whether the increase of the NH₂ 20-22 kDa human tau truncated fragment was also correlated to the pathological changes of full-length tau-such as phosphorylation, conformation and aggregation-, underlying the evolution of AD neurofibrillary tangles (NFTs). To this aim, synaptic-enriched fractions were probed with different antibodies that detect some relevant AD-like epitopes -i.e. AT180, PHF13, AT100, AT8 (2I-L-M-N) and Alz50 epitopes (not shown), which correlate with the PHFs stages progression of the disease [89]. As shown in fig. 2I-L-M-N and in contrast to the decline of several synaptic markers, the immunoreactivity of PHFs-tau changes appeared to be markedly increased in synaptosomes from diseased cases and no comparable intensity band was detected in age-matched control, in line with previous studies from others [44,90]. In addition, an accumulation of HMW tau species -as result of protein aggregation prior to its assembly into highly insoluble PHFs- was also found with specific phosphorylation-dependent (fig.2I-L-M-N-), as well as with phosphorylation independent-(fig.S1) tau antibodies.

The immunoreactivity intensity of the NH2-derived tau fragment in AD synaptosomes correlated with the increase of active caspase-3 (fig.2O) and with the pathological phosphorylation of α-synuclein at Ser-129 (fig.2P), which is found to coexist in several familial Alzheimer's disease (FAD) pedigrees with senile plaques, at synaptic terminals of degenerating neurons containing hyperphosphorylated tau [90-93].

Finally, as shown in fig.2Q-R, the presence of the 4KDa Aβ peptide and the N-terminal 29kDa fragment of the gamma-site-APP-cleaving enzyme (PS1) [94] was also checked in synapses-enriched fractions from controls and AD patients, by probing with 6E10 (residues 4-9 of human Aβ) and NH₂-PS1 (residues 21-80 of human PS1) secretase antibodies respectively.

These findings show that the distribution of NH₂ 20-22 kDa truncated tau fragment is directly correlated with synaptic decay associated to AD neuropathology.

### The solubility profile of the NH₂ 20-22 kDa tau fragment

Tau becomes progressively insoluble during AD stages and filamentous tau aggregates isolated from diseased brains are hallmarks of tauopathies, such as AD [95]. The mechanism underlying the conversion of tau protein from a soluble state to one of insoluble aggregates still remains elusive, but the pathological post-translational modifications of protein -i.e. site-specific hyperphosphorylation and truncation- are considered several of the precipitating pro-aggregation events [96]. Therefore, the authors investigated the solubility profile of the NH₂ 20-22 kDa tau fragment by sequential extraction with buffers of increasing stringency [56] from human AD and control synaptosome-enriched fraction, followed by Western blotting with NH₂4268 tau antibody (residues26-36 of human tau). As shown in fig.3 and quantified by densitometric analysis reported below, a large amount of NH₂-truncated tau fragments doublet was extracted by high-salt-reassembly buffer (RAB) and the immunoreactivity intensity was higher in AD cases (3,15 fold) than in controls. In contrast, only the lower 20-22 kDa resolved band was recovered in detergent-soluble RIPA fraction and a significantly larger portion was detected in diseased (5,12 fold) compared to non-demented control samples. Moreover, such highly insoluble NH₂-derived tau form was recovered in 2%SDS buffer only in AD (4,05 fold), but it was totally absent in control fraction.

These data show that the fast migrating 20-22 kDa band of the NH₂-tau doublet exhibits a different solubility profile compared to the larger fragment, since the former is also extracted with higher stringency buffers, suggesting its possible multimerization/aggregation in NFTs with the AD pathology progression.

### The NH₂ 20-22 kDa tau fragment is localized in AD synaptic mitochondria

The synaptic terminals of polarized cells, such as differentiated neurons, are filled of mitochondria necessary to support the local energy demand [97-99] and the deficiency or impairment of such organelles might cause synaptic dysfunction in cellular and animal AD models [100,101]. In view of these considerations, the authors performed several biochemical and morphological studies in AD as well as in control cerebral hippocampal tissues to assess whether the NH₂ 20-22 kDa tau fragment that the authors detected in human synapse(s) was also present in mitochondria.

To this aim, the authors first carried out the biochemical separation of cytosolic and mitochondrial protein fractions isolated from AD and non-demented control cerebral hippocampus, followed by an immunoblotting analysis. As shown in fig.4A, the upper band of the NH₂-truncated doublet was mainly recovered in cytosolic fraction, while the fast migrating 20-22 kDa band was extracted for the most part in mitochondrial fraction and, more importantly, at higher level in AD than in control samples. Notice also that, in AD samples, the immunoreactivity reduction of the slower migrating band detected in cytosolic fraction was inversely correlated to the net increase of intensity signal of faster band in mitochondria. This finding also suggested that the increase of the NH₂ truncated 20-22 kDa tau fragment was almost totally limited to synaptic mitochondrial fraction.

The efficiency of homogenate fractionation and the purity of mitochondrial preparation was checked by probing fractionated protein extracts with several specific antibodies reacting with cytoplasmatic (copper/zinc-Cu/Zn-SODI) and mitochondrial markers (manganese-Mn-SODII, cytC, COXIV, ANT). As shown, the immunoreactivity intensity of ANT (fig.4B) and COXIV (fig.4C) -other two mitochondrial proteins both localized instead to inner membrane of organelle- was much stronger in mitochondria-enriched fractions, while faint traces were detected in cytosol in AD as well as in non-demented control samples. On the contrary, the immunoreactivity intensity of cyt C (fig.4D) and MnSOD-II (fig.4E) -two soluble mitochondrial proteins localized to intermembrane space and matrix respectively- was surprisingly high in cytosol probably, due to mechanical rupture during organelle purification procedures or to cytosolic release of its apoptogenic factors-i.e. cyt C (102,103). However, the authors rule out that the NH₂ truncated 20-22 kDa tau fragment detected in the mitochondrial fraction is because of non-specific binding of the protein to the surface of the outer membrane, since Cu/ZnSOD-I (fig.4F) -a protein exclusively present in cell body- was totally found in cytosolic extracts and it was completely absent in mitochondria-enriched fractions. In addition ANT (fig.4B) and COXIV (fig.4C), which are membrane-bound proteins confined to the organelle inner compartment, were not recovered in the cytoplasmatic fraction, further demonstrating the purity of their mitochondrial preparations.

The preferential accumulation of the lower NH₂ -truncated 20-22 kDa human tau fragment in AD mitochondria was also related to an impairment of organelle metabolism, as shown by marked decrease of COXIV (fig.4C) and cyt C, MnSOD-II (fig.4D, E) which have known to be deregulated in affected patients [104-112]. On the contrary, an increase of ANT expression level (fig.4B) -probably as result of non-functional compensatory response by the surviving neurons for the loss of other mitochondrial markers [100,113-116]- was contextually observed. Furthermore, no significant difference was detected in the cytosolic Cu/Zn-SOD-I (fig.4F) among AD and age-matched control fractions.

Several studies have reported that APP and Aβ peptide species are associated with mitochondria isolated from both brains and synaptosomal fractions [117-122] of human and AD transgenic mice, probably by direct interaction with specific mitochondrial proteins [105,123-125]. Therefore the authors checked their mitochondrial preparations by probing with 6E10, an antibody reacting with residues 4-9 of human Aβ peptide. As shown in fig.4G, the authors found that monomeric 4kDa Aβ peptide and 12kDa trimer were present in AD biopsies and they were largely enriched in mitochondria. On the contrary, no 4kDa Aβ peptide species were detected in cytosolic, as well as in mitochondrial fractions, from age-matched non-demented controls.

Next, given that the 20-22kDa NH₂ -truncated human tau fragment is preferentially distributed at human synapse(s), the authors tested its actual subcellular localization in synaptic-enriched mitochondria. As shown in fig. 4H-I-L, immunoblot analysis confirmed that the expression level of this NH₂ truncated tau fragment was significantly increased in synaptosome-derived mitochondria from AD and that it was negatively correlated to the organelle physiological function. The comparable expression level of mitochondrial-specific HSP70 (fig.4M) between control and AD fractions ruled out the possibility that the immunoreactivity differences were due to loading of different total protein amounts.

To further dissect the 20-22 kDa NH₂ human tau fragment in association with mitochondria, the authors carried out electron microscopy studies with gold-conjugated NH₂ 4268 antibody on cerebral hippocampal biopsies. As shown in fig.4N-O, the immunogold staining showed a preferential sub-synaptic labeling inside AD mitochondria (fig.4O), with no uniform distribution along matrix and cristae. Furthermore several immunogold-positive particles were also localized at outer and inner membrane, raising the possibility that the intracellular pool from the N-derived D25-cleaved human tau fragments might exhibit different localizations in the diseased mitochondria. Quantification showed that approximately 10 and 214 gold particles for ND and AD mitochondria were present, respectively. No significant labeling was detected in age-matched non-demented control mitochondria (fig.4N) and the specific staining pattern disappeared when the primary antibody was omitted (data not shown). It is noteworthy to notice that, athough the NH₂ 4268 antibody used in this experimental procedure was previously preadsorbed with full-length human tau protein, the authors cannot rule out that the immunogold-staining was exclusively related to the N-truncated D25-tau fraction and not also due to its incomplete affinity-purification. Nevertheless, as previously demonstrated, since a consistent part of 20-22 kDa NH₂ tau fragment was strongly detected on mitochondrial fraction upon biochemical sub-fractionation and Western blotting analysis on AD synaptosomes (fig.4H) and no significant labeling was contextually detected in nondemented samples with pre-adsorbed gold-conjugated NH₂ 4268 antibody (fig.4N), the authors argued that the immunoelectron-positivity in AD images was almost totally limited to the intracellular population of N-derived D25-cleaved human tau fragments.

Taken together, these results demonstrated that the sub-synaptic concentration of the 20-22 kDa NH₂ human tau fragment is higly-enriched in AD mitochondria.

### The soluble extracts containing Aβ oligomers from AD brain homogenates generate the NH₂ 20-22 kDa tau fragment and impair the mitochondrial function in human differentiated SY5Y.

Synthetic Aβ oligomers evoke in human cortical neurons an acute (12-24 hours) impairment of mitochondrial oxido-reductase activity, which was accompanied by the ATP intracellular and by the caspases 3 and 7 activation [102]. Similar results were also found in primary hippocampal neuron upon treatment with cell-derived soluble oligomer of human Aβ peptides used at low concentration (0.5nM), comparable to that detected in human brain and in CSF [126]. In addition, Aβ oligomeric-induced neurotoxicity may be mediated in rat organotypic hippocampal slice culture, at least in part, through the activation of the ERK1/2 signal transduction pathway, which in turn leads to the caspase-3 dependent tau cleavage [74]. In light of these previous results from others and since the authors revealed in synaptosomes from AD subjects the presence of the NH₂ 20-22 kDa fragment in tight relation to Aβ species, the authors investigated whether soluble extracts from human AD brain -which contain native oligomeric Aβ aggregates [57,60]- could generate *in vitro* the NH₂ 20-22 kDa fragment and affect the mitochondrial metabolic activity. To test this hypothesis dbAMPc/NGF-differentiated human SH-SY5Y human neuroblastoma cells were incubated with 1mg/ml F12-extracted supernatants from AD brain homogenates- as well as from non-demented controls- for 12 h, in the presence or in the absence of 100µM Z-VADfmk or 20µM MDL28170, a pan-caspase(s) or calpain-I inhibitor respectively.

The presence of naturally occurring Aβ oligomers in the preparations was first checked by Western blotting analysis under nondenaturing (native) conditions with A11-a polyclonal antibody, which recognizes a generic backbone epitope common to the oligomeric state independently of the primary protein sequence [127]- and with 6E10 -a non-conformation-dependent Aβ monoclonal antibody. Pan-oligomeric A11 antiserum, which only reacts with Aβ oligomers larger than tetramers [127], revealed multiples of oligomers at molecular masses theoretically around 50-75kDa only in crude soluble AD homogenates. These Aβ multimers were also recognized by anti human-specific 6E10 antibody (human Aβ 4-9) (fig.5A, B thin arrows). Alternatively 6E10-positive lower species (fig.5B, thick arrows), which showed only a faint immunoreactivity with A11 antibody, might be to due to the existence of two types of soluble Aβ conformations, which have known as prefibrillar (A11positive) and fibrillar oligomers and are immunologically different [128]. However, the ability of 22C11 antibody (aa 66-81 of APP NH₂-terminus) and 4G8 antibody (Aβ 17-24) (not shown) to recognize similar bands suggest that they were neither APP NH₂-end products, or fragments by soluble APP, which lacks the mid-domain of Aβ epitope recognized by such antibody. Furthermore, the evaluation of Aβ oligomers in crude soluble brain extracts was also performed under denaturating conditions, by SDS-PAGE and Western blotting with 4G8 antibody (Aβ 17-24) (not shown).

Next, by reacting whole cell extracts with NH₂ 4268 tau antibody, the authors detected a significant increase of a 20-22 kDa immunoreactive band in neuronal cultures exposed for 12-14 h to soluble AD extract. Similar results were found upon incubation with synthetic 10µM human Aβ 1-42 peptide (fig.5C) -which contained a mixture of SDS-stable monomer, dimer, trimer/tetramers (fig. S2)- and with 2.5 mM syntethic, human Abeta 1-42 oligomers referred to as ADDLs (not shown). No band of comparable intensity was detected when neurons were incubated with soluble supernatants from control brain homogenates, or with vehicle or inhibitors alone (not shown). In line with the authors' previous paper [46] and as shown in fig. 5C, D-E, the pre-incubation of cultures with Z-VADfmk -and at less extention with MDL28170- significantly reduced the appearance of 20-22 kDa NH₂ tau fragment (fig.5C-D), by blocking the caspase-3 activation (fig.5E). Notice that, upon pharmacological treatment, the immunoreactivity increase of upper band of the NH-2 tau doublet inversely correlates to the reduction of smaller 20-22 kDa intensity signal (fig.5C), suggesting that that the latter fragment represents a proteolytic processing intermediate. In addition and more importantly, the immunoreactivity decrease of mitochondrial COXIV, whose reduction in the expression level has been reported to really correlate with the *in vivo* decline of enzymes activities [105, 107] and that was found in neurons after 12h treatment with soluble AD extracts (fig.5F), is largely prevented by the pharmacho logical inhibition of the NH₂ 20-22Da tau peptide. Finally, as shown in fig. 5G, the pre-incubation of AD brain extracts with A11 -and particularly with 6E10 antibody-significantly reduced the increase of 20-22 kDa NH₂ tau intensity signal in an antibody-dependent manner, demonstrating that NH₂-tau cleavage was, at least in part, mediated by Aβ peptide(s) oligomers in AD. Therefore, it cannot be ruled out that the neurotoxic effect of AD soluble homogenates is not exclusively related to Abeta oligomers. Morevoer, since the 20-22 kDa NH₂ tau immunoreactivity was induced by culture exposure with synthetic, human Aβ1-42 oligomers (fig.5C) and-on the contrary- was reduced with pre-adsorbed NH₂ 4268 antiserum in a 6E10-sensitive manner (fig.5G), the authors argued that the biological effect of the AD soluble extracts was, almost in part, due to Aβ peptide(s). Similar results were also obtained in primary cultures of mature rat hippocampal neurons (21DIV).

The authors' finding suggests that soluble AD extracts containing Aβ oligomeric species can *in vitro* impair the mitochondrial function, likely also through the generation of an NH₂ 20-22 kDa tau fragment.

### The NH₂ 20-22 kDa tau fragment is common hallmark of other human, not-AD tauopathies and in AD patients its amount correlates with the presence of Aβ assemblies and with the pathological severity of disease

The authors investigated whether the NH₂ 20-22 kDa human tau fragment occurred in other tauopathies, which exhibited no or limited Aβ deposition. To this aim, the authors compared the synaptic distribution of this NH₂-derived tau fragment in cases from AD, DLB -which is the second more frequente cause of dementia in the elderly- and PiD - which is is another uncommon tauopathy which differs from AD mainly for tau isoforms composition (3R isoforms) and filaments arrangement [129].

Interestingly, by Western blotting of synaptosomal protein fractions with NH₂ 4268 tau and cleaved caspase-3 antibodies respectively (fig.6A-B), the authors detected an high level of the NH₂ 20-22 kDa tau fragment in correlation to the significant caspase-3 up-regulation, in all diseased cases analyzed but not in control. The extensive NH₂ 4268 tau-positive immunoreactivity found at synapses in examined tauopathies was consistent with other two hallmarks of NFTs pathology -hyperphosphorylation and aggregation-, as shown by probing synapse(s)-enriched protein extracts with AT8 (fig.6D) and PHF13 (fig.6E) antibodies. In contrast to tight association between hyperphosphorylation and NH-2tau truncation, the accumulation of 6E10-positive Aβ species was not correlated with the NH₂ 20-22 kDa tau fragment, in tested non-AD tauopathies (fig-6C lane 6-7). In addition, only Aβ oligomeric assemblies -i.e dimers (8kDa) and especially trimers (14kDa)- generated the NH₂-derived tau fragment in analyzed AD subjects (fig. 6C lane 2-4-5), in correlation to the strong caspase-3 activation. This finding suggest that, in AD, the production of this NH₂-derived tau fragments is likely caspase-3-dependent and mediated by 4kDa Aβ peptide multiples. Finally and more interestingy, although the majority of samples clustered together within their clinically and pathologically group, one sample (lane 3), which was from a young patient (49 years old) suffering of mild/moderated AD, did not. The NH₂ 20-22 kDa tau fragment and active caspase-3 were faintly detected in synaptosomes from this young patient-since their immunoreactivity levels were similar to control non-demented sample- and only the monomeric Aβ peptide was present.

All these data suggest that the amount of NH₂ 20-22 kDa tau fragment correlates with the presence of Aβ multiples species, with caspase(s) activation and with the pathological severity in AD brains.

### The 20-22 kDa NH₂ tau fragment co-immunoprecipitates together with ANT-1 in AD synaptic mitochondria

The authors recently reported that a caspase-derived kDa NH₂-truncated tau fragment, which is likely found between 26 and 250 aminoacids of the longest full-length tau isoform and migrates around 20-22kDa, was largely enriched in synaptic mitochondria from AD and 3 month-old Tg2576 brains. Its amount in terminal fields correlated with the pathological synaptic changes, with the presence of Aβ multimeric species and with mitochondrial functional impairment [174] due to the inhibition of the Adenine Nucleotide Translocator (ANT-1)-mediated ADP/ATP exchange [49] In view of the above findings, the authors sought to investigate whether the synaptic 20-22 kDa NH₂-truncated tau fragment actually *in vivo* interacts with ANT-1 in AD mitochondria and its potential deleterious implications on organelle metabolism.

To this aim, the authors first generated a neo-epitope antiserum (Caspase-Cleaved Protein-NH₂ 4268 tau antibody 26-36 residues -herein termed CCP-NH₂ tau antibody) recognizing the human tau protein truncation at D25, a known N-terminal caspase(s)-cleavage site previously identified in cellular and animal AD models [46] and in human AD brains [175]. The data from analyzed human brain specimens of clinically and neuropathologically confirmed AD cases are summarized in Table 1. As shown in figS3A, a NH₂-tau fragment of 20-22kDa-herein termed NH₂htau- but not-full-length protein running at 55-75kDa, was efficiently immunoprecipitated by CCP-NH₂tau antiserum in experiments on hippocampal synaptic-enriched fractions from AD patients. No band of comparable intensity is detected in age-matched not-demented controls. Note also that-by probing with such antibody -no significant cross-reaction was detected with intact human tau proteins, that were only faintly detected in total extracts from 6h STS-treated neuronal SY5Y and synaptosomal proteins lysates from AD subjects.

Next the authors analyzed whether ANT-1-the neuronal-specific isoform of ANT protein-was a mitochondrial target of the NH₂htau fragment. Co-immunoprecipitation experiments on synaptic-enriched fractions -which contain the complete presynaptic terminal, including mitochondria and synaptic vesicles, along with the postsynaptic membrane and the postsynaptic density (PSD)- were performed from AD and ND cases using CCP-NH₂ tau antiserum as bait-antibody, followed by immunoblotting with a monoclonal detection-antibody which specifically interacts with the least C-terminal 12 amino acids of human neuronal ANT-1(Leung et al.,2008) ; ANT1: ADP/ATP translocase 1 [Homo sapiens]-Accession number NP_001142:

As shown in fig.S3C, ANT-1 co-imunoprecipitated with the NH₂htau fragment, suggesting that the intact ANT-1/NH₂htau complex was present in the synaptosomes from AD brains. On the contrary, in age-matched, not-demented control brains with undetectable or low levels of NH₂htau fragment, there was virtually no detection of ANT-1 signal, suggesting that formation of ANT-1/NH₂htau complex was associated with cerebral levels of NH₂ htau peptide and therefore was excluded from ND mitochondria. No signal was found when the bait-antibody CCP NH₂tau was replaced by preimmune IgG (not shown). Cyt C (fig.S3D) and MnSOD-II (fig.S3 E) -two soluble mitochondrial proteins localized at the intermembrane space and matrix, respectively- were not immunoprecipitated by CCP- NH₂ tau antibody. The accumulation of the NH₂ htau fragment in AD mitochondria was also related to an impairment of organelle metabolism, as shown by marked decrease of cyt C, which is known to be deregulated in affected patients (Parihar et al.,2007). On the contrary, the observed increase of ANT-1 expression level (fig.S3C) was probably as result of non-functional, mitochondrial compensatory response by the surviving neurons to brain injury [215;213] or to an increased oxidative damage [211] although its chronic up-regulation is reported to ultimately activate the apoptotic mitochondrial permeability transition pore (mtPTP) and to lead to neuronal death (Bauer et al.,1999). In a similar way, α-synuclein, a neuronal protein which binds the C-terminal domain of tau (Jensen et al.,1999; Giasson et al., 2003) and is constitutively present in mitochondria of rodents and human and whose mitochondrial accumulation contributes *in vivo* to the decreased activity of complex I in neurodegeneration [197;198], was also not contextually pulled-down in the NH₂-tau immunoprecipitates (fig.S3F).These findings demonstrated the specificity of the protein-protein interaction, thus arguing that the ANT-1/NH₂htau binding was not caused by nonspecific cellular injury. Control experiments demonstrated that a NH₂ htau fragment was efficiently enriched by immunoprecipitation with the respective antibody (fig.S3B).

Since cellular and mitochondrial integrity may start to deteriorate soon after death allowing nonphysiological interactions to occur, the authors evaluated the specificity of such protein-protein interaction by isolating mitochondrial fractions from diseased and age-matched not-demented human synaptosomes. The purity of the mitochondrial preparation was confirmed by the enrichment of specific organelle markers and by the relative absence of cytosolic proteins, as previously reported [174]. Next the authors carried out a reciprocal immunoprecipitation, with the immunocapture mouse ANT-1 antibody, to test for the presence of the NH₂ htau fragment. A shown in fig. S3H, IP/Western blotting analysis further corroborated that a part of the intracellular NH₂ htau fragment stably interacts *in vivo* with mitochondrial ANT-1. Control experiments demonstrated that ANT-1 was efficiently immunoprecipitated by the respective antibody (fig.S3G). Similar results were also obtained with another ANT-1 antiserum (45-233 aminoacids of human ANT-1) (not shown) and in differentiated human SH-SY5Y cells undergoing apoptosis following 6h staurosporine treatment and in 24h NGF-deprived hippocampal neurons (not shown). Densitometric analysis of signal intensity of immunoreactivity bands generated from all co-immunoprecipitation results (AD=3;ND=1;6h SY5Ytotal protein extracts=3) revealed that ANT1/20-22kDa NH₂tau fragment complexes from synapses were increased by 2.5 fold in AD compared to ND.

### The NH₂htau fragment is associated to ANT-1 in AD mitochondria

It has been reported that pathological tau[218]-as well as Aβ [223]- induce an altered anterograde mitochondrial trafficking by causing their accumulation in the soma and paucity in distal regions of neurons [222] which, in turn, indirectly induces the "synaptic starvation" with the local depletion of the energy demand. An impaired balance of fission/ fusion and mitophagy towards enhanced fission [221] may also account for the mitochondrial morphological alterations found in several areas of AD patients [222]

In view of these findings, the authors ascertained whether the interaction of NH₂htau with ANT-1 in an Aβ-rich environment may be also correlated to an altered morphology and/or sub-cellular distribution of mitochondria by comparing them on sections of AD and age-matched, ND brains. By confocal double-immunofluorescence with CCP-NH₂ tau and ANT-1 antibodies shown in fig.S4, a qualitative morphological analysis revealed that an high proportion of NH₂htau fragment-positive structures colocalized with mitochondrial ANT-1 expression in AD cerebral cortices, along a pronounced increase in the immunofluorescence intensity for both markers. On the contrary, a different, non-overlapping distribution was found in age-matched, ND tissues.

In ND tissue, the intracellular pool from the NH₂ human tau fragments was detected at low- intensity by CCP-NH₂ antibody and the immunoreactivity was diffusely distributed into the cytoplasm with a rather occasional grainy staining, barely distinguishable from the surrounding tissue background. Of note, the CCP-NH₂ tau antibody appeared faintly and selectively to stain neurons and not glial cells. These positive neurons showed a rounded or triangular central nucleus and a regular cytoplasmatic shape (Fig.S4B, arrows), while putative glial cells- which are intensely stained for DAPI-were almost devoid of immunoreactivity. On the contrary, the mitochondrial ANT-1 immunoreactivity was characterized by a medium intensity, punctate distribution which was regularly and extensively diffused in the entire tissue parenchyma and appeared to enrich all the cellular compartments of both neurons and glial cells (Fig.S4C, arrows), in absence of any obvious co-staining with CCP-NH₂ tau antibody(Fig.S4D). Specific staining was lost by omission of CCP-NH₂ tau and ANT-1 antibodies or by replacement of specific antibodies pre-adsorbed with their respective antigens or by preimmune IgG or (not shown).

In AD tissue, the expression level of NH₂ human tau fragments was detected at high-intensity by the CCP-NH₂ antibody in swollen, tangle-like neurons, characterized by the protruding nucleus- which was often flattened on one side of the cell body- and by the irregularly shaped cytoplasm (Fig.S4F, arrows). Importantly, the CCP-NH₂tau and ANT-1 antibodies showed an almost complete co-localization in morphologically abnormal, clustered mitochondria which were constricted in the soma (Fig.S4H, arrows). Only few ANT-1-immunopositive structures appeared devoid of CCP-NH₂ tau immunoreactivity, while all structures CCP-NH₂tau-immunopositive presented ANT-1 immunoreactivity. In addition and as shown by comparative analysis with DAPI staining (Fig.S4E), the NH₂tau/ANT-1 immunoreactive structures were observed mainly in putative neurons, while the single ANT-1 immunoreactivity was also detected in glial cells. As reported [222], while in ND tissue the typical ANT-1 immunopositivity was diffuse and distributed along the perikarion and fibers with a characteristic staining of mitochondrial proteins, in diseased cases the NH₂tau/ANT-1 immunoreactive mitochondria were intensely stained and appeared to be segregated at one side of the globose cytoplasm. The perinuclear clustering, retraction of mitochondria and tau-tagged mitochondria toward the cell center- shown in fig.2G- may reflect the neuronal traffic jamming, likely due to the Ab-induced microtubule destabilization [223] and to the loss of extreme N-terminal tau domain which is directly involved in axonal transport by binding to the C-terminus of the p150 subunit of the dynactin complex (Magnani et al., 2007) and to ATP depletion (Escobar-Khondiker et al.,2007).

Although mitochondria were mainly redistributed away from axons showing a reduced density in processes in AD brains [222] and since neurons may lose processes in sections due to the angle of cutting in both AD and control cases- which may mask the possible mitochondria differences at synapses- the authors decided to visualize these organelles at the periphery of neurons towards the terminal fields. Interestingly, the authors found a similar co-staining pattern between the NH₂ htau fragment and ANT-1 away form DAPI-positive nuclei, only in diseased tissues. An abnormal mitochondrial morphology towards enhanced fission [221] was also detected, suggesting an impaired organelle dynamism in AD neurons (fig.S4P).On the contrary, no overlapping immunoreactivity between the NH₂ htau fragment and ANT-1 and /or abnormal mitochondrial morphology were visualized in the cell periphery of control neurons (fig.S4L).

Taken together with the biochemical data of reciprocal co-immunoprecipitations on synaptic-enriched mitochondrial fractions, these morphological studies provide further evidence that the NH₂ htau fragment specifically binds to mitochondrial ANT-1 in AD neurons and corroborate our hypothesis that this relevant disease-related tau alteration might induce the *in vivo* synapses decay also by directly disturbing the physiological organelle functions on the ANT-1-mediated ATP/ADP exchange.

### The 20-22kDa NH₂tau fragment is present in human CSF from patients affected by tauopathies, including AD.

It is accepted that the protein tau is a necessary mediator of Aß-induced neurotoxicity in AD [28] and that, more importantly, the N-terminal half of protein is the active death domain [235]. In Alzheimer's disease (AD), both hippocampal volumes (HVs) and cerebrospinal fluid (CSF) tau markers are associated with neurofibrillary tangles deposits: (1) antemortem HVs assessed by using magnetic resonance imaging (MRI) volumetry significantly correlates with the density of neurofibrillary tangles at autopsy but not with amyloid beta protein (Aβ) plaque load, and (2) CSF tau levels correlated with the presence of neocortical neurofibrillary tangles.

In addition, the increased levels of hyper-phosphorylated tau protein (but not with CSF Aβ42 and T-tau) are reported as specific indicator of the AD progression and different N-terminal fragments have been also detected in CSF of incipient AD patients [186 ,41] and their different patterns may reflect disease-specific neurodegenerative processes [176]. Extracellular tau is toxic in human neuroblastoma and in primary cultures of hippocampal and cortical neurons [177] and the N-terminal of human tau is specifically secreted to the extracellular space and to adjacent neurons in situ tauopathy model [178]. In addition, the up-take [232]and the trans-synaptic transfer of tau-mediated toxicity [233]have been also described in cultured cells and in transgenic mice brain. These findings suggest that truncated tau forms- released into extracellular space as result as neuronal damage/death or active secretion -might also exert their toxic action outside of neurons

The *in vivo* immunization with pospho-tau also slows down the tangle-related behavioural phenotype, suggesting the importance of developing a separate, alternative tau-directed therapy for AD[234]. Finally, since Aβ and tau pathologies are synergistic and Aβ reduction alone isn't sufficient to improve the AD-related cognitive decline[28,33] targeting both not only should be essential for an early, more precise diagnosis but also should increase the treatment efficiency.

Interestingly, the authors observed that a valuable 20-22 kDa NH₂terminal tau-fragment was largely enriched in human synaptosomes of AD brains. This tau-fragment was endogenously produced by cultured neurons when exposed to Aß oligomers and it impaired the mitochondrial functions [174] and it was toxic *per se* when overexpressed in cultured neurons [47,48]. Here, the authors report that this NH₂-terminal tau peptide is largely present in the CSF of AD patients-as well as in CSF subjects affected by other tauopathies- in relation to the classical neurophathological hallmarks. In detail, the authors show that a specific,valuable, toxic [46,48] NH₂tau fragment- migrating at 20-22 kDa of molecular weight- is found (i) in CSF from AD patients, in correlation to degree of dementia (because its amount increases from MCI to middle-late AD cases) and to the pathological phosphotau/Aβ levels (ii) in CSF from subjects carrying other tauopathies (i.e. FTD, PD). On the contrary the amount of this NH₂-derived tau peptide is undetectable in CSF from healthy, age-matched controls.

As shown (fig.7), results on levels of total/phospho- tau and Aβ1-42 in CSF of patients with probable AD- or other tauopathies- confirmed previous findings using methodological standardization in the CSF assay. On the contrary, there were no significant differences in age, and CSF albumin or albumin ratio (CSF albumin (mg/L)/serum albumin (g/L)) among the patient groups(data not shown).

The analysis of the total tau demonstrated a significantly high level in the CSF of AD patients (median: 568.0pg/mL, *n* =20) compared with non demented patients (median:213.5pg/mL, *n* = 16) and with the group of other dementias (median:247.0pg/mL, *n* =13) and with the group of PD (median:133.0pg/mL, *n* = 16). The very high SD in the latter group was particularly due to the presence of the cases with suspected Creutzfeldt-Jacob disease (total CSF tau > 1300pg/mL). The analysis of the phospho- tau(Thr181) also demonstrated a significantly high level in the CSF of AD patients (median 61pg/mL, *n* =20) compared with non demented patients (median:29.5pg/mL, *n* = 16) and with the group of other dementias (median.35.0pg/mL, *n* = 13) and with the group of PD (median:19.0pg/mL, *n* = 16)

At the same time, the levels of Aβ1-42 were significantly lower in the AD demented (median: 251.0 pg/mL; *n* =20) compared with the values in non demented group (median: 520.0 pg/mL; *n* = 16), and with the values in the group of other dementias (median: 410.0pg/mL, *n* =13) and of PD (median:370.0pg/mL, *n* = 16).

Next, the biochemical identification of the NH₂ 20-22kDa tau fragments in CSF was investigated. 500µl -1000µlCSF from single patient was concentrated and analyzed by SDS-PAGE. After electroblotting, the filters were probed with CCP-NH₂ 4268 tau, an antibody that only recognizes human tau protein truncated at D25 residue without any significant reactivity against the full length protein [46]. As shown in fig.7, in line with the significant, increased total/phospho-tau and decreased Aβ1-42 levels found by ELISA in AD cases, an high level of 20-22kDa NH₂ tau fragment was also detected in diseased subjects but not in age-matched, healthy controls (around 4.5 fold, p< 0.001) even when the blots were overexposed for quite a long time. To determinate whether NH₂-truncation of tau in CSF was due to proteolysis that occurred subsequent to samples collection, CSF aliquots were collected by lumbar puncture from each patients directly in protease inhibitor cocktail and immediately frozen. SDS-PAGE analysis showed an identical proteolytic NH₂ 20-22kDa fragment by CCP-NH₂ 4268 tau antibody,suggesting that its presence was not due to experimental handling.To verify that tau NH₂ proteolysis did not occur after the CSF was collected, 10 µg of recombinant human tau (h40, 441 aa) was added to aliquots of CSF from AD and control patients and immunoprecipitated with T46 antibody, an antibody that reacts against the C-terminal of full-length tau protein. Immunoblotting with CCP-NH₂ 4268 antibody of T46-immunoprecipitates showed that only intact tau was recovered and no NH₂- fragment of protein was contextually found. No band of 20-22kDa molecular weight was detected by the omission of primary CCP-NH2 4268 tau antiserum or when it was replaced by an equal amount of the rabbit immunoglobulins, even when the filter was overexposed for a long times. In addition, although the detection of the toxic 20-22kDa NH₂tau fragments in CSF couldn't be used for differential diagnosis for human tauopathies -because it was also found in subjects carrying other tauopathies even if at much lower level than in AD cases -the authors remarked that (i) it was an NH₂ tau fragment whose presence was strictly diseases-linked and was not caused by nonspecific cellular injury (because it increased around 4,5 fold in diseased cases and it was absent in healthy, age-matched controls) and that (ii) its quantification is correlated to the severity of AD pathology as evaluated by temporal patterning of CSF from middle-late AD and early MCI (converters and non-converters).

In view of these findings the authors concluded that (i) the neurotoxic [47,48] NH₂tau fragment-migrating at 20-22 kDa of molecular weight- was specifically and largely present in human CSF from AD patients-as well as in subjects carrying other tauopathies- but non in age-matched, healthy controls(fig.7) and that (ii) its quantitative evaluation in CSF could provide a novel, more specific biomarker for their diagnosis/prognosis in clinical practice.

### DISCUSSION

As previously shown [174] by biochemical fractionation and morphological analysis the authors reported that a significant proportion of 20-22 kDa NH₂-derived human tau fragment- that was shown to be *neurotoxic in vitro-* was preferentially located in the synaptic-enriched mitochondria from AD hippocampus and frontal cortex, when compared to age-matched non-demented controls. The authors' data also showed that the *in vivo*, prominent and preferential synaptic distribution of this NH₂ tau peptide was directly linked to the extent of neurofibrillary degeneration, to the amyloid neuropathology and, more importantly, to the mitochondrial impairment and the synapse(s) degeneration in human AD brains. The authors also detected that (i) the crude soluble AD extracts, which were largely enriched of Aβ oligomeric species, *in vitro* induced this NH₂-tau cleavage and that its pharmachological inhibition ameliorated the Aβ-dependent mitochondrial impairment in differentiated human SH-SY5Y and that (ii) this NH₂-truncated tau specifically bound ANT-1 in synaptic mitochondria of AD subjects. In the present invention, the authors show that the 20-22 kDa NH₂-truncated tau fragments is also present in the CSF of patents carrying tauopathies- including AD- in relation to the classical neurophatho logical hallmarks. By the present invention, the authors provide a novel, valuable, diagnostic/prognostic tool that should be improve the precision level of current biological tests on CSF from patients affected by tauopathies, especially AD.

### The NH-2 tau truncation is linked to synaptic dysfunction in AD

Synapse decay, that secondarily leads to dying-back neuropathy in affected neurons, is an early hallmark in AD pathogenesis, since it occurs before or even in the absence of neuronal death [9] and provides the best neurobiological correlate of mental impairment during life [1,2]. A growing number of experimental works suggests a causative role of soluble oligomeric species of Aβ peptide(s) in AD synaptotoxicity [75,130-135]. On the other hand, recent evidence shows that pathological tau modifications are an obligatory step to the processes leading to neurodegeneration since, although Aβ peptide(s) can precede and promote tau pathology, its toxicity is also tau-dependent [27,28,136]. NH₂-derived tau peptides are early detected in cerebrospinal fluid of AD patients [40,41] and following traumatic brain injury (TBI) or transient forebrain ischemia [137]. Tau fragments-generated by cleavage at both ends of human protein- have been also found within soma and proximal dystrophic neurites in human AD brain, frequently around Aβ-immunoreactive plaques [35-37,138] and the presence has been directly correlated with the extent of neuropathology and with the cognitive decline, during the progression of the disease [36,139]. Tau cleavage has been identified in living Tg4510 mice -which reversibly express P301Lmutant human tau [96,140]- and in other tauopathies, such as Pick's Disease (PiD), cortico basal degeneration (CBD), dementia with Lewy Bodies (DLB) and progressive supranuclear palsy (PSP) [141].

Truncated tau species have been previously found in a *C*. *elegans* model of tauopathy, which exhibits defects in cholinergic synaptic transmission [142]. A direct link between tau hyperphosphorylation and synaptic changes is supported by recent studies reporting that an early phospho-Ser396 tau immunoreactivity has also been found in synaptic-enriched fractions of frontal cortex from AD and DLB biopsies, in association to pathological Ser129 α-synuclein phosphorylation [90]. Cytometry and immunohistochemical methods reveal that a pathological accumulation of Aβ peptide and tau hyperphosphorylation (AT100;12E8;AT8;PHF1;AT180;MC1) occurs concomitantly, within synaptic terminals [43,44] in brain sections from human AD subjects and from Tg2576 and 3XTg AD mice models. Finally, htau -a transgenic mouse line in which the endogenous tau gene is replaced by the wild-type human gene- develops forebrain tau pathology, without any sensorimotors deficits [143], and shows an age-dependent learning impairment involving the synaptic dysfunction [144]. However, of note, none of these previous studies from others have focused on the possible localization of such truncated tau forms also in synaptic terminal fields.

It is noteworthy to notice that *in vivo* immunohistochemistry studies have previously assessed the sub-synaptic distribution of the N-derived D25-cleaved human tau fragments reporting that CCP-NH₂ tau antiserum [35] strongly labels NFTs, neuropil threads, and dystrophic neurites in hippocampal sections from AD brain, while no staining is detected in age-matched controls. A great degree of labeling with the same CCP-NH₂ tau antibody was also reported in neuropil of synaptic fields in the hippocampus of AD transgenic mice [46]. Nevertheless and in a similar way of the *in vivo* expression pattern of active caspase-3 [70], the authors' biochemical data indicated the prominent presence of the 20-22 kDa NH-2- tau fragment in the highly purified synaptic-enriched fractions. However, the finding that a fraction of 20-22 kDa NH₂-derived tau fragment is detectable by biochemical sub-fractionation and Western blotting analysis of synaptosomes indicates that the concentration of this fragment is highly enriched in synaptic compartment, although it cannot be ruled out that it is also present at lower level - undetectable by the authors' protocols- in other neuronal compartments. This is in line with the fact that tau is an axonal microtubule-binding proteins (MAP) whose C-tail binds the cytoskeleton while the N-terminal domain associates with plasma-membrane [145]. The strong intensity signal which segregated with specific synaptic markers (i.e. PSD95) found in synaptosomes-enriched protein extracts from AD human brains and the requirement of long time exposition to detect an appreciable band in diseased total proteins extracts (fig.1E-F; S1) further strengthen the preferential localization of the 20-22 kDa NH₂ tau fragment in synapses from AD patients. Finally, the authors rule out the possibility that the synaptic distribution of the the 20-22 kDa NH₂ tau fragment in AD cases was simply an artifact of PMI, of the subcellular fraction procedure or of the type of analyzed disease/dementia for several reasons. First, there was no significant correlation between these pathological changes and PMI (which was very short, about 3-4 hours). Second, as shown in one young AD case (fig.6 lane 3), at early stages of disease the synaptic level of the N-derived tau fragment was undetectabl, likely due intrinsic biological variability, in concomitance with the parallel reduction of local caspase-3 activation and the absence of Aβ peptide(s) oligomers. Third, the positive correlation between the caspase(s) activation, the pathological NH-2 tau truncation and hyperphosphorylation is also found in other Aβ-independent genetic tauopathies affecting different brain regions. On the other hand and more interestingly, the direct correlation observed in human AD synaptosomes between the active caspase(s) [70], the disease-related synaptic alterations and the NH₂ 20-22 kDa tau fragment, strongly suggest that (i) its *in vivo* formation is likely caspase(s)-mediated and that (ii) it might contribute to the synaptic dysfunction.

It is also important to consider that, although the full length, not-phosphorylated tau was contextually detected in the authors' synaptic fractions, its level was unchanged. Nevertheless, the authors cannot rule out a possible role of phosphorylated full- length tau in synaptic modifications [90] so that the functional interrelationship between total vs truncated tau and their contribution to the death in AD remains to be further investigated. Moreover , although the authors indicate that caspase(s) may be the main protease(s) responsible in the generation of the 20-22 kDa fragment [46], the authors do not exclude the possibility for an involvement of calpain in the generation of this fragment (fig. 5C). On the other hand, the relative roles of caspase(s) and calpastatin-calpain in tau proteolysis [38,45] is supported by a growing body of evidence delineating a complicated cross-talk between the two proteolytic systems in different neuronal culture models [72], upon treatment with Abeta [61]. In addition since (i) neurons containing caspase-3 active not necessarily undergo acute death ([140] and (ii) active caspase-3 and caspase-derived tau fragment (Asp421) colocalize in AD brain [36,37] the authors suggest that, even though NH₂-truncated tau is not an early event, it could however contribute to the progression of the AD disease.

The use of more selective inhibitors, mutational analysis and/or mass spectrometry studies might confirm the exact *in vivo* location of the N-terminal caspase(s)-truncation site of human tau, while a more large screening of different diseased samples might help us to better clarify the precise timing of NH₂-tau truncation. Finally, the mechanism by which truncated N-tau is concentrated at synapses in pathological conditions still remains to be investigated.

### The mitochondrial distribution of NH₂ 20-22 kDa tau peptide may exacerbate the synapses degeneration in AD

The synaptic terminals are high energy-demanding sites since they require a continuous ATP supply. In order to support a constant ATP bioavailability, the mitochondria are concentrated at synapses [97] and the changes in organelle functions may affect the synaptic plasticity [99]. Really, strong evidence indicates that the synaptic mitochondrial dysfunction plays a crucial role in the AD pathogenesis [98-100] and that the paucity of mitochondria causes the synaptic dysfunction in dendrites and axons [146,147]. Ultrastructural mitochondrial changes -which correlated with the loss of neuritic arborization and with the pathological alteration of dendritic spines- has been described in AD brains, in comparison with the normal controls [148,149]. Aβ peptide(s) may bind mitochondria in AD patients, as well as in transgenic AD mice models [105,117,118,122,123,150], altering its physiological functions [105,120,151-154]. In addition several studies demonstrate that Aβ species are present in mitochondrial synaptosomal fractions in association with the organelle swelling, the depletion of synaptic vesicles and the reduction of glucose metabolism [121,155], suggesting therefore that Aβ peptide(s) damages synapse(s) in AD neurons, likely by acting on residing mitochondria. The present study show that (i) the NH₂ 20-22kDa tau fragment is localized in the synaptic AD mitochondria, in correlation to the organelle dysfunction, (ii) the soluble aqueous extracts enriched of Aβ oligomers from AD brains generate this NH₂ tau peptide, in an antibody-sensitive manner, and disturb the mitochondrial function, in mature human neurons, (iii) the blockade of this NH₂tau cleavage ameliorates the Aβ-dependent *in vitro* mitochondrial dysfunction. Taken together with the previous cited studies, the authors' data suggest that the energy metabolism of synaptic mitochondria might be impaired by Aβ oligomeric species through multiple cellular mechanisms, also involving the *in vivo* NH₂-tau cleavage. In addition, the synaptic localization of this N-derived tau fragment in other hereditary tauopathies, which are characterized by limited Aβ deposition and by the mitochondrial impairment [156], suggest that several Aβ-independent stressors could likely negatively impact on the local mitochondria function, through a common mechanism involving the NH₂-tau cleavage.

Regarding the possible impact of truncated tau forms on mitochondrial function, it has been reported that neurons derived from rat transgenic animals expressing truncated [151-391] human tau acquired an *in vivo* neurofibrillary AD pathology [157] and exhibited a significant reduction of the mitochondria number and their altered distribution in processes which, in turn, caused the accumulation of ROS, sensitizing cultures to cell death induced by oxidative stress [158]. Proteomic and functional analysis of brains from P301L tau transgenic mice, which exhibit caspase-3 activation and caspase-3-mediated tau cleavage into smaller intermediate fragments [159], revealed a marked mitochondrial dysfunction-with no variation of organelle number- in correlation to synaptic alterations [160]. Moreover, as reported in a recent study [161], the inducibile expression of caspase(s)-truncated Asp421 tau in cortical neurons evoked a strong mitochondrial fragmentation, probably through activation of calcium-dependent phosphatase calcineurin, suggesting that caspase(s)-mediated tau cleavage could release one or more fragment(s) that might *in vivo* compromise the mitochondrial function during AD progression. In several NFTs-bearing neurons form AD brains, truncated Asp421 tau fragment, caspase(s) and cyt C were found tightly associated [39]. Intraneuronal accumulation of ATP synthase α-chain -an inner mitochondrial membrane protein of complex V of oxidative phosphorylation- has been also detected in tau-positive NFTs in degeneration AD neurons [162]. As the authors previously showed [49], a synthetic NH₂ 26-44 tau peptide- which is probably included in the NH₂ 20-22 kDa tau fragment analyzed in this work and was the minimal active moiety that retained the *in vitro* marked toxic effect of the longer NH₂-26-230 form [48]- impaired the oxidative phosphorylation, by acting on mitochondrial ANT-mediated ADP/ATP change. Finally, Aβ peptide species and tau protein synergistically impair mitochondrial function in vitro [153] and in vivo [113,114], probably by acting at different level of cellular respiration. Therefore the present invention, which provides for the first time a likely causal link between synaptic mitochondria dysfunction and a NH₂- tau fragment in human AD brains, suggest that disease-related tau alterations might induce the *in vivo* synapses starvation throught two concomitant mechanisms: (i) by depleting locally the energy demand, as an indirect consequence of an altered mitochondrial distribution [158,163,164] and also (ii) by direct disturb of the physiological organelle functions. To this regard, a novel class of antioxidants -including MitoQ, MitoVitE, MitoPBN, MitoPeroxidase, N-acethyl-L-cysteine [165-168] that cross the Blood Brain Barrier (BBB) and specifically target mitochondria- represents a promising approach for AD therapy. Indeed, Dimebon- a mitochondria stabilizing drug- has the ability to improve the cognitive performance in AD patients for at least 12-18 months [169]. Finally, since Aβ-targeted therapeutics in Phase III clinical trials have not given promising results [170,171], the present data induce to consider the *in vivo* NH-2 tau cleavage inhibitors as an alternative drug discovery strategy for AD therapy.

### The NH₂ htau fragment is present in association with mitochondrial ANT-1 in AD synapses

Previously, the authors demonstrated that the synthetic NH₂26-44 tau, which was the minimal active moiety retaining the *in vitro* deleterious effect of longest overexpressed NH₂-derived human tau fragment(s) [48], reduced the intracellular bioavailability of ATP synthesized by neuronal mitochondria[49]. On the contrary NH₂1-25 tau fragment, which was not toxic when overexpressed in neurons[48], had no significant effect on oxidative phosphorylation [49]. By biochemical and immunohistological analysis,here the authors show- for the first time- that a toxic NH₂derived fragment specifically co-immunoprecipitates with ANT-1 in AD synaptic mitochondria. The present invention helps to elucidate the mechanism(s) by which a NH₂-truncated tau form might facilitate synapses deterioration during neurodegeneration and, in particular, how this AD-relevant pathological modification of tau may directly cause synaptic mitochondrial dysfunction by inducing a functional ANT-1 impairment.

Morphological, biochemical, genetic *in vitro* and *in vivo* studies have also revealed that mitochondrial dysfunction is a trigger and/or propagator of neurodegeneration since their functional impairment in human dementias could render selective, vulnerable neurons intrinsically more susceptible to cellular aging and stress and overlying genetic variations [206]. In particular, the impairment of mitochondrial oxidative phosphorylation - that has been extensively documented in the brain of AD patients - is proportional to the clinical disability [190]. Furthermore, synaptic mitochondria- which localize at neuronal, high energy-demand terminal fields and whose damage compromises all physiological cerebral functions [189 ;219; 204] selectively undergo elevated oxidative stress during aging [188], show high level of CypD [212]and are more susceptibile to calcium insult [191].

More recently, a growing evidence suggests that mitochondrial dysfunction might integrate the two AD hallmarks - plaques and the NFTs - which can act on its metabolism independently or synergistically, as well as directly or indirectly. In transgenic AD mice and in AD brains, the monomeric and/or fibrillar Aβ forms localize to mitochondria [205; 193; 196; 194] and directly interact with different mitochondrial proteins -such as ABAD (Lustbader et al.,2004), CypD[200] and probably ANT-1 [216] by damaging them and by causing de-regulation of enzymes, ROS production, inhibition of electron transport chain and ATP production, alteration of mitochondrial trafficking/distribution and dynamism [206 ,193; 217; 192 ; 211; 207; 215]. In transgenic mice overexpressing the mutated human APP, synaptic mitochondria show an early and greater Aβ accumulation in relation to a significant decline of mitochondrial metabolism and an increase of oxidative stress- if compared to nonsynaptic mitochondria [201]. In addition, the inducibile expression of another caspase-truncated tau fragment (Asp-421) in immortalized cortical neurons induces calcineurin-dependent, mitochondrial fragmentation and membrane damage which does not occur in similar, full-length tau isoform-expressing *in vitro* cultures [214] An early mitochondrial impairment with local energy deprivation and lack of spines has been also found in tau-missorted hippocampal dendrites after treatment with Aβ [223] and well as in APP transgenic mice. Finally, an aggravated impairment of oxidative phosphorylation has been also proved in a transgenic AD mice -which coexpress P301Ltau,APPswe,PS2 -if compared with control mice overexpressing APP or tau alone [114], as well as in brains and cerebellum of another triple AD models carrying APPswe/PS1M146V/tauP301L [195]. Taken together these findings suggest that effects of pathological Aβ and tau may converge *in vivo* on mitochondria [203;199] at synapses [209; 208] The present invention provides an important mechanistic link between the pathological NH₂-tau form and the mitochondria impairment involved in early, AD synaptic deterioration. By the analysis of human brain samples, the authors found that (i) a synaptic NH₂-truncated tau-whose minimal active region-i.eNH₂26-44 *- in vitro* inhibited the ANT-1-mediated ADP/ATP exchange in a non-competitive manner[49]- interacts in human AD mitochondria with this protein. Taken together the authors' morphological, biochemical and functional data support a direct cause-effect relationship between the ANT-1/NH₂tau interaction and mitochondrial dysfunction in AD and provides additional insights how tau dysfunction - in parallel to traffic jams of mitochondria[218;223] directly causes their impairment at AD synapses by binding ANT-1. To this regard it is important to notice that the inhibition of the pathological ABAD-Aβ interaction at mitochondria, using a decoy peptide (DP) *in vitro* and *in vivo* has been recently proved to be neuroprotective by defending against aberrant mitochondrial and neuronal function and by improving the spatial learning/memory in transgenic APP mice [223] On the other hand, it has also recently clarified that other factors, including direct mitochondrial dysfunction caused by mutant and/or aggregated protein rather than its impaired mobility, may play a more significant role in the onset of neurodegeneration[224].

### The 20-22kDa NH₂tau fragment is present in human CSF from tauopathies: a valuable, more specific diagnostic/prognostic tool for clinical practice

The crucial role of tau protein in the pathogenesis of tauopathies, especially the AD, has prompted several work aimed at assessing its diagnostic accuracy. Early detection is vital in the quest to develop a cure and CSF biomarkers (Aβ1-42,t-tau,p-tau) can be used to distinguish diseased subjects from healthy controls. Indeed, the neuropathological hallmarks could be reflected in the CSF and these biomarkers are suitable to follow disease progression and to monitor drug intervention due to the correlation with its severity. Sensitive ELISA assays allow tau to be quantified in CSF and studies have shown a significant elevation of CSF levels of tau in AD compared with elderly normal controls and with patients of other neurological disorders [184,40,183,185,187]Increased CSF levels of tau are also found during the AD progression , including in very mild cases. In addition even normal subjects have measurable levels of CSF tau, suggesting that its release in CSF must be considered a physiological process.Moreover, the search for specific NH₂truncated forms of tau in CSF from patients carrying tauopathies was currently under investigation. In this study, the authors developed a Western blot-based approach followed by quantitative evaluation with CCP-NH2 4268 antibody in order to better identify and characterize the NH₂-proteolytic tau fragment(s) in CSF. This approach was indeed preferred to the ELISA analysis, which did not lead to clear-cut findings of all NH₂-derived tau fragments in human CSF[40]. The authors showed that the novel, specific, valuable 20-22kDa NH₂- proteolytic tau fragments could be considered a disease-linked biological phenotype in human tau-related disorders (fig.7), claiming for its diagnostic/prognostic use for clinical practice.

To date, the nature of full-length and truncated tau in CSF has not been well documented. The CSF-tau full-length isoforms migrating at 52 kDa and 65-80 kDa normally occur in both CSF and postmortem brain. Previous studies have reported results for the molecular weight of full-length tau in the CSF in AD, with variations such as 68 kDa,[183] 55 kDa[184] and three bands of 50-65 kDa[185]. In addition, truncated tau forms have been also documented, with one band of 26-28 kDa [186] in lumbar CSF and several bands in the range of 30-50 kDa in ventricular CSF[187]. The methodological differences including the use of different tau antibodies, the absence of any neuropathological diagnoses combined to fewer controls and the lack of detailed quantitative/comaparative analysis further complicate the credibility and the accurate clinical interpretation of these discrepant results. In addition, considering that (i) the low concentrations of tau in the CSF (300ng/L in healthy individuals and 900ng/L in AD patients) and that (ii) its distribution over many different modified forms and splice variants make its detection difficult, it would have been uncertain to detect this low-abundance, neuron-specific protein by Western blot in few µl of unmodified CSF. Indeed CSF tau protein can be detected by using much larger, concentrated CSF volumes or by using sensitive detection techniques such as enzyme-linked immunosorbent assay (ELISA) or mass-spectrometry [41]. In the present work, several experimental procedures that made clear that the NH₂-derived 20-22 kDa tau fragment and no other, aspecific bands were visualized- were taken by authors: (i) the monitoring of the amount of tau in CSF by ELISA and the preselecting of samples with relatively large amount of protein, the replicating of experiments, the controlling for confusing factors such as proteolysis or human immunoblotting cross-reaction, the running of appropriate controls and standard within every experiments.

It is noteworthy that others authors detected a larger NH₂-tau fragment of 26-28kDa-including epitope between 19-46 of longest human tau- by immunoprecipitation of pooled AD CSF samples- but they didn't identify the potential cleavage-site(s) on full-length protein. Interestingly and in line with our previous paper [174], the authors also found that (i) the NH₂ 4268 tau antibody recognized by Western blotting a similar immunoreactive doublet ranging between 20 and 30 kDa in synaptosomes from AD but not in normal elderly controls and that (ii) the fast migrating resolved band was more abundant than the slower in AD (n=15) but not in control (n=10) (around 9-10 fold higher). As previously reported (fig.1), the authors suggested that the larger NH₂-derived tau fragment [40] could be generated from cleavage at the two nearby caspase(s) sites in the NH₂tau domain- i.e. caspase-3 and -6 - or could be represented a post-translational modification of a single smaller NH₂tau fragment -i.e ubiquitination/phosphorylation. Alternatively, since the larger of the two proteolytic fragments had a stronger signal on Western blot in correlation to faint immunoreactivity of the smaller, it could be possible that the latter fragment represented a proteolytic processing intermediate.

In conclusion, by the present invention, the authors propose that the dynamic evaluation of the NH₂-derived 20-22kDa tau form(s) by temporal pattering of CSF might add some hints in the clinical practice of human tauopathies ,thus providing an adjunctive marker for theirs diagnosis/prognosis.

### REFERENCES

[1] Terry RD, et al. (1991) Ann. Neurol 30, 572-80.
[2] DeKosky ST, Scheff SW (1990) Ann Neurol 27, 457-64.
[3] Masliah E, et al. (2001) Neurology 56, 127-9.
[4] Masliah E, Crews L, Hansen L (2006) J Alzheimers Dis 9, 91-9. Review.
[5] Scheff SW, Price DA, Schmitt FA, Mufson EJ (2006a) Neurobiol Aging 27, 1372-84.
[6] Scheff SW, Price DA (2006b) J Alzheimers Dis 9, 101-15. Review.
[7] Terry RD (2000) J Neuropathol Exp Neurol 59, 1118-9.
[8] Selkoe DJ (2002) Alzheimer's disease is a synaptic failure. Science 298, 789-91.
[9] Coleman PD, Yao PJ (2003) Neurobiol Aging 24, 1023-7. Review.
[10] Pomponi M, Bria P, Pomponi M (2008) J Alzheimers Dis 13, 39-47. Review.
[11] Almeida CG, et al. (2005) Neurobiol Dis 20, 187-98.
[12] Lanz TA, Carter DB, Merchant KM (2003) Neurobiol Dis 13, 246-53.
[13] Jacobsen JS, et al. (2006) Proc Natl Acad Sci U.S.A.103, 5161-6.
[14] Rutten BP, et al. (2005) Am J Pathol 167, 161-73.
[15] Scheff SW, Price DA (2003) Neurobiol Aging 24, 1029-46. Review.
[16] Scheff SW, et al. (2007) Neurology 68, 1501-8.
[17] Masliah E, et al. (1994) Neurosci Lett 174, 67-72.
[18] Gylys KH, et al. (2004) Am J Pathol 165, 1809-17.
[19] Honer WG (2003) Neurobiol Aging 24, 1047-62. Review.
[20] Kojima N, Shirao T (2007) Neurosci Res 58, 1-5. Review.
[21] Reddy PH, et al. (2005). J Alzheimers Dis 7, 103-17; discussion 173-80.
[22] Mukaetova-Ladinska EB, et al. (2000) Am J Pathol 157, 623-36.
[23] Blanpied TA, Ehlers MD (2004) Biol Psychiatry 55, 1121-7. Review.
[24] Knobloch M, Mansuy IM (2008) Mol Neurobiol 37, 73-82. Review.
[25] Harigaya Y, Shoji M, Shirao T, Hirai S (1996) J Neurosci Res 43, 87-92.
[26] Shim KS, Lubec G (2002) Neurosci Lett 324, 209-12.
[27] Rapoport M, et al., (2002) Proc Natl Acad Sci U. S. A. 99, 6364-9.
[28] Roberson ED, et al. (2007) Science 316, 750-4.
[29] King ME, et al. (2006) J Cell Biol 175, 541-6.
[30] Götz J, Chen F, van Dorpe J, Nitsch RM (2001) Science 293, 1491-5.
[31] Lewis J, et al. (2001) Science 293, 1487-91.
[32] Oddo S, et al., (2003) Neurobiol Aging 24, 1063-70.
[33] Oddo S, Billings L, Kesslak JP, Cribbs DH, LaFerla FM (2004) Neuron 43, 321-32.
[34] Gouras GK, et al (2000) Am J. Pathol 156, 15-20.
[35] Rohn TT, et al. (2002a) Neurobiol Dis 11, 341-54.
[36] Rissman RA, et al. (2004) J Clin Invest 114, 121-30.
[37] Gamblin TC, et al. (2003) Proc Natl Acad Sci U. S.A. 100, 10032-7.
[38] Park SY, Ferreira A (2005) J Neurosci 25, 5365-75.
[39] García-Sierra F, et al., (2008) J Alzheimers Dis 14, 401-9. Review.
[40] Johnson GV, et al. (1997) J Neurochem 68, 430-3.
[41] Portelius E, et al. (2008) J Proteome Res 7, 2114-20.
[42] Wang YP, et al. (2007) Proc Natl Acad Sci US. A. 104, 10252-7.
[43] Fein JA, et al. (2008) Am J Pathol 172, 1683-92.
[44] Takahashi RH, (2008) Neurobiol Aging In press.
[45] Canu N, et al. (1998) J Neurosci 18, 7061-74.
[46] Corsetti V, (2008) Mol Cell Neurosci 38, 381-92.
[47] Amadoro G, et al. (2004) Cell Death Differ 11, 217-30.
[48] Amadoro G, et al. (2006) Proc Natl Acad Sci U. S. A. 103, 2892-7.
[49] Atlante A, et al. (2008) Biochim Biophys Acta 1777, 1289-300.
[50] McKhann G, et al. (1984) Neurology 34, 939-44.
[51] Mirra SS, et al.(1991) Neurology 41, 479-86.
[52] Wemmie JA, et al. (2002) Neuron 34, 463-77
[53] Cho KO, Hunt CA and Kennedy MB (1992). Neuron 9, 929-942.
[54] Kim YJ, et al. (2001) Proc Natl Acad Sci U. S. A. 98, 12784-9.
[55] Matsuo ES, et al.(1994) Neuron 13, 989-1002.
[56] Zhukareva V, Trojanowski JQ, Lee VM (2004) Am J Geriatr Psychiatry 12, 136-145.
[57] Gong Y, et al. (2003) Proc Natl Acad Sci U.SA. 100, 10417-10422.
[58] Tokuyasu, K.T (1973) J Cell Biol 57, 551-565.
[59] Culmsee C, et al. (2002) Neuroscience 115, 1089-108.
[60] DeFelice FG, et al. (2008) Neurobiol Aging 29, 1334-47.
[61] Wei Z, et al. (2008) Neuropharmacology 54, 721-33.
[62] WB Stine Jr, et al. (2003) J Biol Chem 278, 11612-11622
[63] Volontè C, Ciotti, MT, Battistini L (1994) Cytometry 17, 274-276.
[64] Glimmer T, et al. (2008) Neurobiol Aging 30, 1902-9.
[65] Li Y, et al. (2008) EurJNucl Med Mol Imaging 35, 2169-81.
[66] Scher AI, et al. (2007) Neuroimage 36, 8-18.
[67] Arendt T (2009) Acta Neuropathol 118, 167-79.
[68] Wyszynski M, et al. (1998) J Neurosci 18, 1383-92.
[69] Dosemeci A, et al. (2006) Biochem Biophys Res Commun 339, 687-94.
[70] Louneva N, et al. (2008) Am J Pathol 173, 1488-95.
[71] Horowitz PM, et al. (2004) J Neurosci 24, 7895-902.
[72] Neumar RW, et al. (2003) J Biol Chem 278, 14162-7.
[73] Yuste VJ, et al. (2002) J Neurochem 80, 126-39.
[74] Chong YH, et al. (2006) J Biol Chem 281, 20315-25.
[75] Lesné S, et al. (2006) Nature 440, 352-7.
[76] Minger SL, et al. (2001) J Neuropathol Exp Neurol 60, 929-36.
[77] Qin S, Hu XY, Xu H, Zhou JN (2004) Acta Neuropathol 107, 209-15.
[78] Perdahl E, et al. (1984) J Neural Transm 60, 133-41.
[79] Davidsson P, Blennow K (1998) Int Psychogeriatr 10, 11-23.
[80] Love S, et al. (2006) Neurobiol Aging 27, 797-803.
[81] Leuba G, et al. (2008) J Alzheimers Dis 15, 139-51.
[82] Gibson PH, Tomlinson BE (1977) J Neurol Sci 33, 199-206.
[83] Heredia L, et al. (2006) J Neurosci 26, 6533-42.
[84] Maloney MT, Bamburg JR (2007) Mol Neurobiol 35, 21-44.
[85] Suzuki T, Okumura-Noji K (1995) Biochem Biophys Res Commun 216, 582-8.
[86] Grant SG, et al. (1992) Science 258, 1903-10.
[87] Shirazi SK, Wood JG (1993) Neuroreport 4, 435-7.
[88] Ho GJ, et al. (2005) Neurobiol Aging 26, 625-35.
[89] Luna-Muñoz J, et al. (2007) J Alzheimers Dis 12, 365-75.
[90] Muntané G, Dalfó E, Martinez A, Ferrer I (2008) Neuroscience 152, 913-23.
[91] Lippa CF, et al. (1998) Am J Pathol 153, 1365-70.
[92] Rosenberg RN (2005) Arch Gen Psychiatry 62, 1186-92.
[93] Leverenz JB, et al. (2006) Arch Neurol 63, 370-6.
[94] Beher D, et al. (1999) J Neurochem 72, 1564-1573.
[95] Goedert M, Spillantini MG, Cairns NJ, Crowther RA (1992) Neuron 8, 159-68.
[96] Spires-Jones TL, et al. (2009) Trends Neurosci 32, 150-9. Review.
[97] Ly CV, Verstreken P (2006) Neuroscientist 12, 291-9.
[98] Parihar MS, Brewer GJ (2007) Am J Physiol Cell Physiol 292, C8-23
[99] Mattson MP, Gleichmann M, Cheng A (2008) Neuron 60, 748-66. Review.
[100] Reddy PH, Beal MF (2008) Trends Mol Med 14, 45-53.
[101] Reddy PH (2009) Exp Neurol 218, 286-92. Review.
[102] Deshpande A, Mina E, Glabe C, Busciglio J (2006) J Neurosci 26, 6011-8**.**
[103] Kim HS, et al. (2002) Neuroreport 13, 1989-93.
[104] Maurer I, Zierz S and Moller HJ (2000) Neurobiol Aging 21, 455-462.
[105] Devi L, et al. (2006) J Neurosci 26, 9057-68.
[106] Pérez-Gracia E, Torrejón-Escribano B, Ferrer I (2008) Acta Neuropathol 116, 261-8.
[107] Vargas T, et al. (2008) Neurobiol Aging In Press.
[108] Gibson GE, Sheu KF, Blass JP (1998) J Neural Transm 105, 855-70. Review.
[109] Cardoso SM, et al.(2004) Neurobiol Aging 25, 105-10.
[110] Cottrell DA, et al. (2002) Neuropathol Appl Neurobiol 28, 390-6.
[111] Marcus DL, et al. (2006) Med Sci Monit 12, BR8-14.
[112] Simonian NA, and Hyman BT (1994) J Neuropath Exp Neurol 53, 508-512.
[113] Rhein V, et al. (2009a) Cell Mol Neurobiol 29, 1063-71.
[114] Rhein V, et al. (2009b) Proc Natl Acad Sci U. S. A. In Press.
[115] Nagy Z, Esiri MM, LeGris M, Matthews PM (1999) Acta Neuropathol 97, 346-54.
[116] Reddy PH (2006) J Neurochem 96, 1-13. Review.
[117] Manczak M, et al. (2006) Hum Mol Genet 15, 1437-49.
[118] Caspersen C, Wang N, Yao J, Sosunov A, Chen X et al. (2005) FASEB J 19, 2040-1.
[119] Yamaguchi H, et al. (1992) Acta Neuropathol 85, 15-22.
[120] Anandatheerthavarada HK, et al. (2003) J Cell Biol 161, 41-54.
[121] Mungarro-Menchaca X, Ferrera P, Morán J, Arias C (2002) J Neurosci Res 68, 89-96.
[122] Crouch PJ, et al. (2005) J Neurosci 25, 672-9.
[123] Lustbader JW, et al. (2004) Science 304, 448-52.
[124] Du H, et al. (2008) Nat Med 14, 1097-105.
[125] Hansson Petersen CA, et al. (2008) Proc Natl Acad Sci U. S.A. 105, 13145-50.
[126] Yang TT, Hsu CT, Kuo YM (2009) J Neural Transm In press.
[127] Kayed R, et al. (2003) Science 18, 486-9.
[128] Kayed R, et al. (2007) Mol Neurodegener 26, 2:18.
[129] Goedert, Jakes R (2005) Biochim Biophys. Acta 1739, 240-50. Review.
[130] Lacor PN, et al. (2007) J Neurosci 27, 796-807.
[131] Lacor PN, et al. (2004) J Neur osci 24, 10191-200.
[132] Koffie RM, et al. (2009) Proc Natl Acad Sci U. S.A. 106, 4012-7.
[133] Shankar GM, et al. (2008) Nat Med 14, 837-42.
[134] Snyder EM, et al. (2005). Nat Neurosci 8, 1051-8.
[135] Shankar GM, et al. (2007) J Neurosci 27, 2866-75.
[136] Sotiropoulos I, et al. (2008) J Neurochem 107, 385-97.
[137] Siman R, et al. (2004) Neurobiol Dis 16, 311-20.
[138] Rohn TT, et al. (2001) Am J Pathol 158, 189-98.
[139] Basurto-Islas G, et al. (2008) J Neuropathol Exp Neurol 67, 470-83.
[140] Spires-Jones TL, et al. (2008) J Neurosci 28, 862-7.
[141] Newman J, et al. (2005) Acta Neuropathol 110, 135-44.
[142] Kraemer BC, et al. (2003) Proc Natl Acad Sci U. S. A. 100, 9980-5.
[143] Andorfer C, et al. (2003) J Neurochem 86, 582-90.
[144] Polydoro M, et al. (2009) J Neurosci 29, 10741-9.
[145] Brandt R, Léger J, Lee G (1995) J Cell Biol 131, 1327-40.
[146] Li Z, Okamoto K, Hayashi Y, Sheng M (2004) Cell 119, 873-87.
[147] Verstreken P, Ly CV, Venken KJ, Koh TW, Zhou Y et al. (2005) Neuron 47, 365-78.
[148] Baloyannis SJ (2006) J Alzheimers Dis 9, 119-26. Review.
[149] Baloyannis SJ (2009) JNeurol Sci 15, 153-7.
[150] Takuma K, Yan SS, Stern DM, Yamada K (2005) J Pharmacol Sci 97, 312-6.
[151] Keil U, et al. (2004) J Biol Chem 279, 50310-20.
[152] David DC, et al. (2005) J Biol Chem 280, 23802-14.
[153] Eckert A, et al. (2008a) J Mol. Med 86, 1255-67.
[154] Eckert A, et al. (2008b) Neurodegener Dis 5, 157-9.
[155] Gillardon F, et al (2007) Proteomics 7, 605-16.
[156] Spillantini MG, Goedert M (1998) Trends Neurosci 21, 428-33. Review.
[157] Zilka N, et al. (2006) FEBS Lett 580, 3582-8.
[158] Cente M, et al. (2006) Eur J Neurosci 24, 1085-90.
[159] Ramalho RM, et al. (2008) Mol. Med 14, 309-17.
[160] David DC, et al. (2005) J Biol Chem 280, 23802-14.
[161] Quintanilla RA, et al. (2009) J Biol Chem 284, 18754-66.
[162] Sergeant N, et al. (2003) Neuroscience 117, 293-303.
[163] Thies E, Mandelkow EM (2007) J Neurosci 27, 2896-907.
[164] Chee FC, et al. (2005) Neurobiol Dis 20, 918-28.
[165] Reddy PH (2006) J Biomed Biotechnol 3, 31372.
[166] Sheu SS, et al. (2006) Biochim Biophys Acta 1762, 256-65. Review.
[167] Szeto HH (2006) A.A.P.S. J. 8, E521-31. Review.
[168] Murphy MP, Smith RA (2007) Annu Rev Pharmacol Toxicol 47, 629-56. Review.
[169] Doody RS, Gavrilova SI, Sano M, Thomas RG, Aisen PS (2008) Lancet 372, 207-15.
[170] Brunden KR, Trojanowski JQ, Lee VM (2009a) Nat Rev Drug Discov 8, 783-93.
[171] Brunden KR, et al. (2009b) Exp Neuro In Press.
[172] Smith CJ, Anderton BH, Davis DR, Gallo JM (1995) FEBS Lett 375, 243-8.
[173] Deshpande A, et al. (2009) J Neurosci 29, 4004-15.
[176] Borroni B, et al. (2009) Neurobiol Aging 30(1), 34-40
[177] Gomez-Ramos A, et al. (2008) Mol Cell Neurosci 37(4), 673-681
[178] W, et al. (2009) J Alzheimers Dis[Epub ahead of print]
[174] Amadoro G, et al. (2010) J Alz.Dis. 21(2):445-70
[179] Rohn TT, et al. (2009) Int J Clin Exp Pathol.2(2):108-18
[180] Sigurdsson EM. (2009)Curr Alzheimer Res. 6(5):446-50.
[183] Wolozin B, et al. (1987) Ann Neurol 22:521- 6.
[184] Vigo-Pelfrey C, et al. (1995) Neurology 45:788-93.
[185] Arai H, et al. (1995) Ann Neurol38:649-52.
[186] Johnson GV, et al. (1997) J Neurochem68: 430-3.
[187] Zemlan FP,et al. (1999) J Neurochem;72:741-50.
[188]Banaclocha MM, et al. (1997) Brain Res 762:256-258
[189] Billups et al. (2002) J Neurosci. 22(14):5840-7.
[190]Blass JP (2003) Neurol. Res. 25 6: 556-566.
[191]Brown MR, et al. (2006) JBiol Chem 281:11658-11668
[192]Cardoso SM, (2004) Neurobiol Aging. 25(1):105-10.
[193]Caspersen C,et al. (2005) FASEB J 19: 2040-1.
[194]Chen JX, et al. (2007) Expert Rev Neurother. 7(11):1517-25.
[195]Ciavardelli D, (2010) Cell Death Dis. 1(10):e90.
[196] PJ, et al. (2002) J Neurosci. 19: 25(3):672-9.
[197]Devi L, et al. (2008) J Biol Chem. 283(14):9089-100.
[198]Devi L, (2010) Biochim Biophys Acta. 1802(1):11-9.
[199]Diana FF, et al. Curr Alzheimer Res. Jan 19. [Epub ahead of print]
[200]Du H, et al. (2008) .NatMed 14(10):1097-105.
[201]Du H, et al. (2010) Proc Natl Acad Sci US A. 107(43):18670-5.
[202]Du H, et al. (2010). Int J Biochem Cell Biol 42:560-572.
[203] A, et al. (2010) Mol Neurobiol 41(2-3):107-14.
[204]Evans RJ, et al. (1992). Nature. 357(6378):503-5.
[205]Fernández-Vizarra P, et al. (2004) .Histol Histopathol. 19(3):823-44.
[206]Galindo MF, et al. (2010) J Neurochem. 114(4):933-45.
[207]Hansson Petersen CA, et al. (2008). Proc Natl Acad Sci U. S. A. 105: 13145-50.
[208]Hoover BR, et al. (2010) Neuron 68(6):1067-81.
[209]Ittner LM, et al. (2010) Dendritic function of tau mediates amyloid-beta toxicity in Alzheimer's disease mouse models.Cell 142(3):387-97.
[210]King ME, et al. (2006) J Cell Biol 175: 541-6.
[211]Manczak M, et al. (2006) Hum Mol Genet 15: 1437-49.
[212] KK, et al. (2007) J Neurosci 27:7469-7475.
[213]Nagy Z, et al. (1999) Acta Neuropathol 97: 346-54.
[214]Quintanilla RA, et al. (2009) J Biol Chem.284(28):18754-66.
[215]Reddy PH, et al. (2010) J Alzheimers Dis.20 Suppl 2:S499-512. Review
[175]Rohn TT, et al. (2002) Neurobio/ Dis 11: 341-54
[216]Singh P, et al.(2009) Bioinformation. 3(10):440-5.
[217]Takuma K, et al. (2005) FASEB J. 19(6):597-8.
[218]Thies E et al. (2007) J. Neurosci. 27: 2896-2907.
[219]Verstreken P, et al. (2005). Neuron. 47(3):365-78.
[220]Wang X, et al. (2008) Proc Natl Acad Sci USA. 105(49):19318-23.
[221]WangX, et al. (2009) J Neurosci. 29(28):9090-103.
[222]Wang X, et al. (2010) Neurodegener. Dis. 7:56-59.
[223]Zempel H, et al. (2010) J Neurosci. 30(36):11938-50.
[223] Yao J, et al. (2011) J Neurosci. 31(6):2313-20.
[224] Zhu YB, Sheng ZH. (2011) J Biol Chem. [Epub ahead of print]
[225]Götz J et al. (2008) Neuropsychiatr Dis Treat. 4(6):1033-42.
[226]Bauer MK, et al. (1999) J Cell Biol. 147(7):1493-502.
[227]Giasson BI,et al. (2003) Science. 300(5619):636-40.
[228]Jensen PH, et al. (1999) J Biol Chem. 274(36):25481-9.
[229]Leung AW, et al. (2008 J Biol Chem. 283(39):26312-23
[230]Magnani E, et al. (2007) EMBO J. 26(21):4546-54.
[231]Parihar MS et al. (2007) Am J Physiol Cell Physiol. 292(1):C8-23.
[232] Frost B (2009) J Biol Chem. 284(19):12845-52.
[233] Clavaguera F et al. (2009) Nat Cell Biol11(7):909-13.
[234] Asuni AA(2007) J Neurosci. 27(34):9115-29.
[235] Ittner LM (2010) Cell.142(3):387-97.

## Claims

1. A method for the diagnosis and/or prognosis of a tauopathy in subject comprising:
- quantifying the amount of a 20-22 kDa NH₂ tau fragment comprising the sequence QGGYT MHQDQEGDTD AGLKESPLQT PTEDGSEEPGSETSDAKSTP TAEDVTAPLV DEGAPGKQAA AQPHTEIPEG TTAEEAGIGD TPSLEDEAAG HVTQARMVSK SKDGTGSDDK KAKGADGKTK IATPRGAAPP GQKGQANATR IPAKTPPAPK TPPSSGEPPK SGDRSGYSSP GSPGTPGSRS RTPSLPTPPT REPKKVAVVR (SEQ ID No. 1) or of a fragment of SEQ ID 1, said fragment missing 1 to 10 amino acids, in a CSF sample obtained from the subject;
- comparing the quantified amount of the 20-22 kDa NH2 tau fragment comprising SEQ ID No. 1 or of said fragment thereof in the CSF sample to appropriate control amount.

2. The method of claim 1 wherein the tauopathy is selected from the group of: Alzheimer's Disease (AD), Dementia with Lewy Bodies (DLB), Pick's disease (PiD), cortico basal degeneration (CBD), or progressive supranuclear palsy (PSP).

## Patentansprüche

1. Verfahren für die Diagnose und/oder Prognose einer Tauropathie in einem Individuum, umfassend:
- Quantifizieren der Menge eines NH₂-tau-Fragments mit 20-22 kDa, umfassend die Sequenz QGGYT MHQDQEGDTD AGLKESPLQT PTEDGSEEPGSETSDAKSTP TAEDVTAPLV DEGAPGKQAA AQPHTEIPEG TTAEEAGIGD TPSLEDEAAG HVTQARMVSK SKDGTGSDDK KAKGADGKTK IATPRGAAPP GQKGQANATR IPAKTPPAPK TPPSSGEPPK SGDRSGYSSP GSPGTPGSRS RTPSLPTPPT REPKKVAVVR (SEQ ID Nr. 1), oder eines Fragments von SEQ ID 1, wobei dem Fragment 1 bis 10 Aminosäuren fehlen, in einer Liquorprobe, die von dem Individuum erhalten wurde;
- Vergleichen der quantifizierten Menge des NH₂-tau-Fragments mit 20-22 kDa, umfassend SEQ ID Nr. 1, oder des Fragments davon in der Liquorprobe mit einer geeigneten Kontrollmenge.

2. Verfahren nach Anspruch 1, wobei die Tauropathie aus der Gruppe Alzheimer-Demenz (AD), Demenz mit Lewy-Körperchen (DLB), Morbus Pick (PiD), kortikobasaler Degeneration (CBD) oder progressiver supranukleärer Blickparese (PSP) ausgewählt ist.

## Revendications

1. Procédé de diagnostic et/ou de pronostic d'une tauopathie chez un sujet comprenant :
- la quantification de la quantité d'un fragment NH₂ de tau de 20 à 22 kDa comprenant la séquence QGGYT MHQDQEGDTD AGLKESPLQT PTEDGSEEPGSETSDAKSTP TAEDVTAPLV DEGAPGKQAA AQPHTEIPEG TTAEEAGIGD TPSLEDEAAG HVTQARMVSK SKDGTGSDDK KAKGADGKTK IATPRGAAPP GQKGQANATR IPAKTPPAPK TPPSSGEPPK SGDRSGYSSP GSPGTPGSRS RTPSLPTPPT REPKKVAVVR (SEQ ID NO : 1) ou d'un fragment de SEQ ID NO : 1, ledit fragment manquant de 1 à 10 acides aminés, dans un échantillon de LCR obtenu du sujet ;
- la comparaison de la quantité du fragment NH₂ de tau de 20 à 22 kDa comprenant SEQ ID NO : 1 ou dudit fragment de celui-ci dans l'échantillon de LCR à une quantité adaptée de témoin.

2. Procédé selon la revendication 1, dans lequel la tauopathie est sélectionnée dans le groupe de : la maladie d'Alzheimer (MA), la démence à corps de Lewy (DCL), la maladie de Pick (MPi), la dégénérescence corticobasale (DCB), ou la paralysie supranucléaire progressive (PSP).
